(19) Europäisches Patentamt
European Patent Office
Office européen des brevets

(11) EP 1 497 309 B1

(12) **FASCICULE DE BREVET EUROPEEN**

(45) Date de publication et mention de la délivrance du brevet:
**14.01.2009 Bulletin 2009/03**

(21) Numéro de dépôt: **03740640.2**

(22) Date de dépôt: **18.04.2003**

(51) Int Cl.:
***C07J 43/00*** *(2006.01)*

(86) Numéro de dépôt international:
**PCT/FR2003/001248**

(87) Numéro de publication internationale:
**WO 2003/089449 (30.10.2003 Gazette 2003/44)**

(54) **COMPOSES AMINOALKYLSTEROLES AVEC ACTIVITE ANTI-TUMEUR ET NEUROPROTECTRICE**

AMINOALKYLSTERINVERBINDUNGEN MIT ANTI-KREBS UND NEUROPROTEKTIVER AKTIVITÄT

AMINOALKYL STEROL COMPOUNDS HAVING AN ANTI-TUMORAL AND NEUROPROTECTIVE ACTIVITY

(84) Etats contractants désignés:
**AT BE BG CH CY CZ DE DK EE ES FI FR GB GR HU IE IT LI LU MC NL PT RO SE SI SK TR**

(30) Priorité: **19.04.2002 FR 0204912**

(43) Date de publication de la demande:
**19.01.2005 Bulletin 2005/03**

(73) Titulaire: **INSTITUT NATIONAL DE LA SANTE ET DE LA RECHERCHE MEDICALE (INSERM)
75013 Paris (FR)**

(72) Inventeurs:
- **POIROT, Marc
F-31240 L'Union (FR)**
- **DE MEDINA, Philippe
F-31130 Balma (FR)**
- **POIROT, Sandrine
F-31240 L'Union (FR)**

(74) Mandataire: **Laget, Jean-Loup et al
Brema-Loyer
161, rue de Courcelles
75017 Paris (FR)**

(56) Documents cités:
- **SHAFIULLAH ET AL: "SYNTHESES OF STEROIDAL SUBSTITUTED TETRAZOLES" ACTA CHIMICA HUNGARICA, vol. 127, no. 3, 1990, pages 391-394, XP008010474 ISSN: 0231-3146**
- **EL KIHEL, L. ET AL: "Synthesis and cytotoxicity of aminosterols: activity studies on a non-small-cell bronchopulmonary carcinoma line (NSCLC-N6)" ANTICANCER RESEARCH (1999), 19(2A), 1229-1234, XP008010475**
- **SUH N ET AL: "A novel synthetic oleanane triterpenoid, 2-cyano-3,12-dioxoolean-1,9- dien-28-oic acid, with potent differentiating, antiproliferative, and anti-inflammatory activity." CANCER RESEARCH. UNITED STATES 15 JAN 1999, vol. 59, no. 2, 15 janvier 1999 (1999-01-15), pages 336-341, XP002220453 ISSN: 0008-5472**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

L'invention a pour objet de nouveaux dérivés de stérols, un nouveau procédé permettant l'obtention de ces composés et l'utilisation desdits composés pour la production de vacuoles sécrétoires dans des cellules tumorales, notamment en vue de la régression de tumeurs cancéreuses, pour augmenter la dendritogénèse et combattre les maladies neurodégénératives et pour l'activation du système immunitaire.

La dendritogénèse est une modification morphologique des cellules qui intervient notamment dans le système nerveux et dans le système immunitaire. Dans le système immunitaire, la dendritogénèse conduit à la génération de cellules dendritiques à partir de monocytes. Ces phénomènes physiologiquement importants ne sont pas bien compris à ce jour, mais ils sont associés à une augmentation de l'activité sécrétoire des cellules (MARTINEZ-ARCA S. et coll., 2001, J. Neurosci, 21 (11), 3830-38 et DENZER K et coll., 2000, J. Cell. Sci., 113 Pt 19, 3365-74). Ces phénomènes sont induits par des facteurs de croissance et des cytokines. La différentiation de monocytes en cellules dendritiques (CD) est induite par le co-traitement des monocytes avec l'interleukine 4 (IL4/GM-CSF), l'activation des CD requérant en outre un traitement au TNF-$\alpha$ (tumor necrosis factor alpha).

Il est connu que les cellules tumorales sont antigéniques et expriment à leur surface des antigènes spécifiquement reconnus par les lymphocytes. Ces antigènes sont des peptides chargés sur les molécules du CMH de classe I : un lymphocyte T $CD8^-$ spécifique reconnaît ce complexe CMH-peptide. On a montré que les CD modifiées ex vivo pour présenter des antigènes tumoraux induisaient une immunité spécifique prévenant et/ou éradiquant des tumeurs établies chez la souris (Schuler P. Steinman RM, J. Exp. Med. 1997, 8, 1183-7 et Angevin, André, Zitvogel, Bull Cancer, 2000, 87(1), 107-15). On a donc considéré que les CD sont des adjuvants puissants pour l'induction d'une réponse immunitaire thérapeutique.

A ce jour, la thérapie cellulaire concerne donc des méthodes pour générer ex vivo des CD humaines à usage clinique.

Dans le cas du cancer du sein notamment, on a déjà proposé de prélever sur le patient des cellules monocytaires et de les cultiver en présence d'une association de cytokines et de l'antigène à présenter ; les cellules dendritiques obtenues par dendritogénèse ont été réinjectées aux patientes pour développer les lymphocytes T cytotoxiques spécifiques de l'antigène (Lawrence Fong et Edgar G. Engleman, Annu. Rev. Immunol., 2000, 18, 245-273).

L'état de la technique, qui vient d'être exposé, met en évidence les inconvénients inhérents à ces méthodes. En effet, la sensibilisation des cellules dendritiques utilise des peptides dérivés d'antigènes tumoraux ; or, dans la majorité des tumeurs, les antigènes spécifiques n'ont pas été identifiés. De plus, pour sensibiliser les cellules dendritiques, on utilise généralement des peptides identifiés dans les cellules tumorales grâce à des clones de lymphocytes T cytotoxiques spécifiques de la tumeur ; or, les épitopes présentés par les cellules tumorales et ceux présentés par les cellules dendritiques présentatrices d'antigène ne sont probablement pas les mêmes. Au surplus, lorsqu'on utilise des cellules dendritiques soumises aux différents traitements ex-vivo ci-dessus évoqués, il peut se produire des changements phénotypiques conduisant à des populations cellulaires hétérogènes peu adaptées à un usage thérapeutique. Il est donc hautement désirable d'améliorer les méthodes d'obtention de cellules dendritiques sensibilisées, pour permettre un développement de l'immunothérapie.

L'invention est basée sur le fait que l'on a constaté que, de façon tout à fait surprenante, l'on pouvait induire in vivo la différenciation de monocytes en cellules dendritiques par une classe de composés nouveaux dérivés de stérols. Jusqu'à présent, l'utilisation de composés non peptidiques n'avait été envisagée que pour potentialiser la neuritogénèse (Pradines et autres, 1995, J. Neurochem., 64, 1954-64) ; rien ne pouvait suggérer à l'homme de métier que des molécules non peptidiques étaient susceptibles d'induire la différentiation de monocytes en cellules dendritiques. Selon l'invention, et de façon tout à fait surprenante, on a trouvé qu'une catégorie de stérols mime, à faible dose, l'effet facteur de croissance dendritogénique et l'effet des cytokines.

La présente invention a, en conséquence, pour premier objet les nouveaux dérivés de stérols susceptibles de procurer l'effet ci-dessus indiqué. Ces dérivés sont les composés de formule (I) :

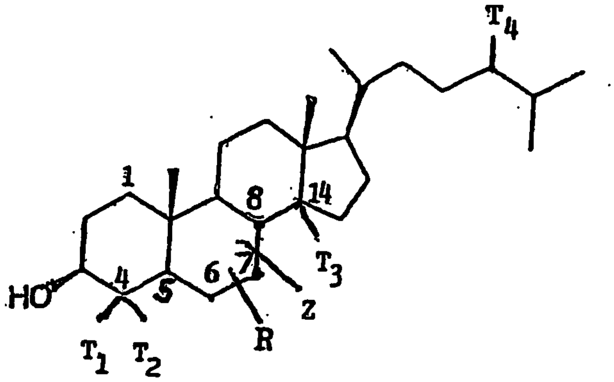

(I)

formule dans laquelle le carbone en position 4 du squelette cholestérol porte des éléments $T_1$, $T_2$, qui peuvent être indépendamment H ou $CH_3$ avec $CH_3$ en $\alpha$ et/ou $\beta$, le carbone en position 24 porte un élément $T_4$ qui représente H, $CH_3$ ou $C_2H_5$, le carbone en position 14 porte un élément $T_3$, qui peut être H ou un $CH_3$ en alpha, l'une des liaisons entre les carbones 5 et 6, d'une part, et 7 et 8, d'autre part, peut être double alors que l'autre est simple, et dans laquelle :

- Z représente, en position 5 ou 8, soit H soit OH, OH ne pouvant être porté que par un carbone qui ne porte pas de double liaison ;
- et R représente en position 6 ou 7, sur un carbone ne portant pas de double liaison, le substituant de formule $-Q_0-Q_1$,

substituant dans la formule duquel
$-Q_0-$ représente le radical de formule (II) :

$$-X-(CH_2)_{n0}\ [Y_1-(CH_2)_{n1}]_{p1}\ [Y_2-\ (CH_2)_{n2}]_{p2}\ [Y_3-(CH_2)_{n3}]_{p3}\ [Y_{4}(CH_2)_{n4}]_{p4}\ [Y_5\ -\ (CH_2)_{n5}]_{p5}-$$ (II)

formule (II) dans laquelle :

. $p_1$, $p_2$, $p_3$, $p_4$, $p_5$ sont des nombres entiers égaux, indépendamment les uns des autres, à 0 ou 1,
. $n_0$, $n_1$, $n_2$, $n_3$, $n_4$, $n_5$ sont des nombres entiers indépendants les uns des autres tels que :

$$1 \leq n_0 \leq 4$$

$$0 \leq n_1, n_2, n_3, n_4, n_5 \leq 4$$

. -X- représente -S-, -O-, $-CH_2-$, $-NR_3-$, où $R_3$ est H ou un radical alkyl en $C_1$-$C_4$, ou encore un hétérocycle

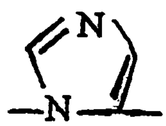

. $-Y_1-$, $-Y_2-$, $-Y_3-$, $-Y_4-$, $-Y_5-$ représentent, indépendamment les uns des autres, -S-, -O-, -C-, $-NR_3-$, où $R_3$ a la signification ci-dessus indiquée ;

et dans la formule duquel

- $Q_1$ représente un noyau indolique, un noyau morpholinique ou thiomorpholinique rattaché par son atome d'azote, un hétérocycle

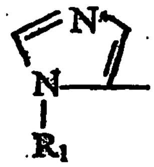

où $R_1$ représente H, $COCH_3$, un radical alkyl en $C_1$-$C_4$, ou encore

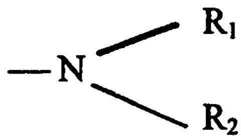

où $R_1$ a les significations ci-dessus indiquées et $R_2$ représente H ou un radical alkyl en $C_1$-$C_4$, $R_1$ et $R_2$ pouvant constituer ensemble un cycle pipéridinique, pyridinique, pipérazinique éventuellement substitué par un radical alkyl en $C_1$-$C_4$, ou encore un hétérocycle pyrolique ou pyrolidinique comportant un atome d'azote et 4 atomes de carbone, sous réserve que :

. si -X- = -NH- et

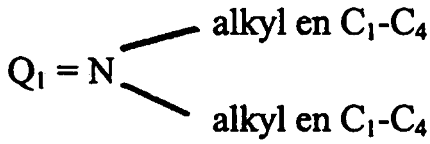

,

l'un au moins des nombres $p_1$, $p_2$, $p_3$, $p_4$, $p_5$ soit différent de 0 ; et
. si -X- = $-CH_2-$, n0 = 1 et tous les nombres $p_1$, $p_2$, $p_3$, $p_4$, $p_5$ sont nuls, $Q_1$ soit différent de $-NH_2$.

La présente invention a aussi pour objet un procédé d'obtention des composés de formule (I) dans lequel :

- dans une première étape, on fait agir l'acide metachloroperoxybenzoïque, dissout dans un solvant A, sur un composé répondant à la formule (III)

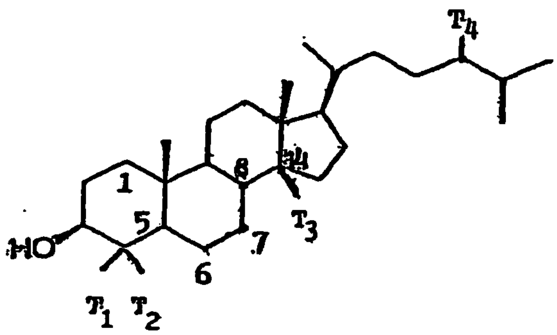

(III)

formule dans laquelle le carbone en position 4 du squelette cholestérol porte des éléments $T_1$, $T_2$, qui peuvent être indépendamment H ou $CH_3$ avec $CH_3$ en $\alpha$ et/ou $\beta$, le carbone en position 24 porte un élément $T_4$ qui représente H, $CH_3$ ou $C_2H_5$, le carbone en position 14 porte un élément $T_3$, qui peut être H ou un $CH_3$ en alpha, l'une au moins des liaisons entre les carbones 5 et 6, d'une part, et 7 et 8, d'autre part, est double, le composé de formule III étant dissous dans un solvant B miscible au solvant A,
- dans une deuxième étape, on fait agir sur le composé époxy obtenu par la première étape, dissous dans un solvant C en présence d'un activateur D, une amine de formule $Q_0Q_1$, $Q_0$ et $Q_1$ ayant les significations indiquées dans la revendication 1, dissoute dans un solvant E miscible au solvant C.

Parmi les composés de formule (I) préférés, il faut citer :

le cholestan-3β, 5α diol 6β-N-[1-*N*1-(3-amino-propyl)-butane-1,4-diamine] ;
le cholestan-3β, 5α diol 6β-N-[*N,N'*-bis-(3-amino-propyl)-butane-1,4-diamine] ;
le cholest-7-ene-3β, 5α diol 6β-N-[1-*N*1-(3-amino-propyl)-butane-1,4-diamine] ;
le cholest-7-ene-3β, 5α diol 6β-N-[*N,N'*-bis-(3-amino-propyl)-butane-1,4-diamine] ;
le cholest-7-ene-3β, 5α diol 6β-N-[2 éthylamino-(3*H*-imidazol-4-yl)] ;
le cholestan-3β, 5α diol 6β-N-[2 éthylamino-(3*H*-imidazol-4-yl)] ;
le cholest-7-ene-3β, 5α diol 6β-N-(4-Amino-butylamine) ;
le cholest-7-ene-3β, 5α diol 6β-N-{2-[2-(2-amino-éthoxy)-éthoxy]-éthylamine} ;
le cholestan-3β, 5α diol 6β-N-[4-(2-amino-éthyl)-imidazol-1-yl] ;
le cholestan-3β, 5α diol 6β-N-({1H-imidazol-4-yl}-éthyl)-acétamide.

On a notamment constaté que l'utilisation des composés de formule (I) à des doses nanomolaires induit la production de vacuoles sécrétoires dans différentes lignes cellulaires tumorales, ce qui, selon l'état de la technique, est généralement associé à une dendritogénèse, et dans des lignées cellulaires immortalisées telles que les NIH-3T3 ou les COS-7.

L'invention a donc aussi pour objet un médicament caractérisé par le fait qu'il comporte, dans un véhicule pharmaceutiquement acceptable, au moins un composé de formule (I).

Le médicament ci-dessus défini est utilisé pour augmenter la dendritogénèse de cellules vivantes de mammifères ; il peut être utilisé pour déclencher une neuritogénèse sur des cellules nerveuses ou leurs précurseurs et pour combattre les maladies neuro-dégénératives humaines.

Le médicament ci-dessus défini est utilisé pour activer le système immunitaire d'un organisme vivant.

Le médicament ci-dessus défini est utilisé pour la production de vacuoles sécrétoires dans des cellules tumorales d'un organisme vivant, notamment pour la régression des tumeurs cancéreuses chez les mammifères.

Le médicament selon l'invention est avantageusement administrable par injection ; dans un tel cas, il peut être utilisé à des doses allant de 8,5 ng/g d'organisme vivant traité à 1,7 μg/g d'organisme vivant traité ; pour le traitement d'une tumeur, l'injection s'effectue, de préférence, à proximité de la tumeur à traiter.

Le caractère surprenant de l'invention provient notamment du fait que des composés ayant une formule voisine de celles définies par la formule (I) n'ont été antérieurement décrites qu'en mentionnant des activités totalement différentes de celles trouvées pour les composés de formule (I) selon l'invention. Dans le brevet français 2 047 880, il est indiqué que les composés décrits ont une activité hypocholestérolémiante. Dans le document "Synthetic Communications (1997), 27(11), 1951-1962", il est indiqué que les composés décrits ont une activité antibiotique. Dans le document "Arzneimittel-Forschung (1995), 45(2), 190-4" et dans le document "Anticancer Research (1999), 19 (2A), 1229-1234", il est indiqué que les composés décrits ont une activité cytotoxique. Il en résulte que l'homme de métier ne pouvait en aucun cas prévoir, à partir de cet état de la technique, que les composés de formule (I) pouvaient induire une dendritogénèse, une neuritogénèse et/ou la production de vacuoles sécrétoires dans différentes lignées cellulaires tumorales.

L'induction de la production de vacuoles a été observée dans différentes lignées cellulaires tumorales et notamment les cellules A549, U937, MCF-7, HT29, PC12 et B16. La dendritogénèse et la neuritogénèse ont été observées sur des cellules tumorales U937 et P19, ainsi que sur des cellules B16 et PC12.

On va donner ci-après plusieurs exemples de préparation des composés selon l'invention. En premier lieu, on va fournir les détails relatifs au procédé commun utilisé pour la préparation des différents composés donnés comme exemples : ce procédé est réalisé en deux étapes, la première consistant en l'obtention d'un époxy-stérol et la deuxième consistant à transformer ledit époxy-stérol en stérol aminé. Les exemples 1 et 2 décrivent chacun la première étape en vue de l'obtention de deux séries différentes de 5,6-α-époxy-stérols. L'exemple 3 décrit la deuxième étape utilisant les produits intermédiaires des exemples 1 et 2 pour les faire réagir avec une amine ; les exemples 4 à 27 décrivent les produits obtenus selon l'exemple 3 avec différentes amines. Les exemples 28 et 29 décrivent chacun la première étape en vue de l'obtention de deux autres séries différentes de 5,6-α-époxy-stérols ; les exemples 30 à 47 décrivent la deuxième étape utilisant les produits intermédiaires des exemples 28 ou 29 pour les faire réagir avec une amine.

<u>Exemple 1</u> : préparation du 5,6-α-époxycholestan-3β-ol :

De l'acide méta-chloro-peroxybenzoïque (0,73 g, 4,25 mmol, pureté de 70-75 % en poids) est dissous dans du chlorure de méthylène (10 ml) et ajouté goutte à goutte à un mélange de cholestérol (1 g, 2,5 mmol) dissous dans du chlorure de méthylène (25 ml). L'agitation est maintenue toute la nuit. Le mélange réactionnel est lavé avec une solution aqueuse de sulfite de sodium (10 % en poids) et d'hydrogénocarbonate de sodium (5 % en poids) et une solution saturée d'un mélange de chlorure de sodium et de chlorure de potassium. La phase organique est séchée sur du sulfate de magnésium anhydre. L'évaporation sous vide du solvant organique permet l'obtention de 0,7 g d'aiguilles blanches

(rendement 69,5 %). La proportion des isomères α et β de l'époxyde a été déterminée par RMN du proton à 200 MHz : on trouve 78 % α époxyde, 22 % β époxyde (RMN du proton 1H : δ 2,89 (d, 1H, J=4,37 Hz, H-6) ; 3,04 (d, J=2,43 Hz, H-6) ; 3,91 (m, 1H, H-3) ; SM DCI/$NH_3$ MH' 403. Les isomères α et β ont été séparés par chromatographie liquide sur silice (toluène/éther éthylique, 85/15). L'isomère α a pour point de fusion pf=141-142°C ; l'isomère β a pour point de fusion pf= 131-132°C. Pour compléter la caractérisation, on a effectué une chromatographie sur couche mince (acétate d'éthyle) ; on a obtenu : Rf = 0,69 (couleur marron après révélation avec le mélange acide sulfurique-méthanol).

Exemple 2 : préparation du 5,6-α-époxycholest-7-en-3β-ol :

Du 7-déshydrocholestérol (Acros, 1 g, 2,6 mmol) et du carbonate de sodium (0,55 g, 5,2 mmol) sont dissous dans un mélange de chlorure de méthylène (25 ml) et d'eau (25 ml). De l'acide méta chloroperoxybenzoïque (0,73 g, 4,25 mmol, pureté 70-75 % en poids) est dissous dans du chlorure de méthylène (10 ml) et ajouté goutte à goutte sur le mélange maintenu sous une agitation vigoureuse. Après 10 minutes d'agitation, la phase est récupérée puis lavée avec une solution aqueuse constituée d'une solution aqueuse de sulfite de sodium (10 % en poids), d'une solution aqueuse de carbonate de sodium (5 % en poids) et d'une solution saturée d'un mélange de chlorure de sodium et de chlorure de potassium. La phase organique est séchée sur du sulfate de magnésium anhydre. L'évaporation du solvant et la recristallisation du produit dans l'acétone permet l'obtention de 0,7 g d'aiguilles blanches (rendement 70 %). La structure a été confirmée par RMN du proton : δ 0,52 (s,3H,H-19) ; 2,986 (d,1H,J=4,1 Hz, H-6) ; 3,91 (m,1H,H-3) ; SM DCI/$NH_3$ $MH^+$ 401. On a déterminé le point de fusion pf = 144-146°C.

Exemple 3 : Synthèse des stérols aminés à partir des époxy-stérols:

Du perchlorate de lithium (0,75 mmol) et une amine sous sa forme basique (1 mmol) sont dissous dans de l'éthanol anhydre (1 ml) et ajoutés sous flux d'argon à une solution éthanolique (3 ml) d'un époxy-stérol obtenu selon l'un des exemples 1 ou 2 (100 mg, 0,25 mmol). Le mélange réactionnel est maintenu sous agitation et à reflux, pendant 6 jours dans le cas de l'exemple 1 et pendant 3 jours en absence de lumière et à température ambiante dans le cas de l'exemple 2. L'avancée de la réaction est contrôlée par chromatographie sur couche mince (CCM) dans les conditions adaptées aux différentes amines. Le solvant est éliminé par évaporation et le résidu est lavé avec de l'éther éthylique (5 x 3 ml) et de l'hexane (5 x 20 ml). Le résidu est solubilisé dans l'eau et acidifié avec HCl 2M (2 ml). La solution est pré-purifiée sur cartouche de silice greffée (sep-pack cartridge RP C18, 500 mg, Waters), l'excès de polyamine est éliminé par passage d'eau sur la cartouche (5 ml). Le produit est élué avec un mélange $CH_2CN$ 1/$H_2O$ 1 (5 ml). Le produit est purifié par HPLC en phase inversée grâce à un gradient linéaire d'un mélange de départ $H_2O$ 95/$CH_3CN$ 5/TFA 0,1 % jusqu'à un mélange $CH_3CN$ 95/$H_2O$ 5/TFA 0.1 atteint en 60 minutes (débit = 1 ml/min ; λ = 210 nm). La fraction d'intérêt a été repurifiée dans des conditions isocratiques en utilisant une phase mobile composée d'un mélange ($CH_3CN$ 95/$H_2O$ 5/TFA 0,1) 44 %/($H_2O$ 95/$CH_3CN$ 5/TFA 0,1 %) 56 % (débit = 1 ml/min ; λ = 210 nm).

Exemple 4 : Préparation du Cholestan-3β, 5α diol 6β-N-[1-*N*1-(3-amino-propyl)-butane-1,4-diamine]

Dans l'exemple 3, on utilise, comme amine, la N1-(3-amino-propyl)-butane-1,4-diamine.

Le produit obtenu est caractérisé spécifiquement par chromatographie sur couche mince (CCM) (alcool isopropylique/ ammoniaque 28 % / $H_2O$:6/3/1) : Rf = 0,62.

On a aussi effectué une spectroscopie de masse (electrospray) : $MH^+$ : 548.

Exemple 5 : Préparation du Cholestan-3β, 5α diol 6β-N-[*N,N'*-bis-(3-amino-propyl)-butane-1,4-diamine]

Dans l'exemple 3, on utilise, comme amine, la N,N'-bis-(3-amino-propyl)-butane-1,4-diamine.

Le produit obtenu est caractérisé spécifiquement par CCM (alcool isopropylique/ammoniaque 28 % / $H_2O$: 6/3/1) : Rf = 0,37.

On a aussi effectué une spectroscopie de masse (electrospray) : $MH^+$ : 605,5.

Exemple 6 : Préparation du Cholest-7-ene-3β, 5α diol 6β-N-[1-*N*1-(3-amino-propyl)-butane-1,4-diamine]

Dans l'exemple 3, on utilise, comme amine, la N1-(3-amino-propyl)-butane-1,4-diamine.

Le produit obtenu est caractérisé spécifiquement par CCM (alcool isopropylique/ammoniaque 28 % / $H_2O$: 6/3/1) : Rf = 0,62.

On a aussi effectué une spectroscopie de masse (electrospray) : $MH^+$ : 546.

Exemple 7 : Préparation du Cholest-7-ene-3β, 5α diol 6β-N-[*N,N'*-bis-(3-amino-propyl)-butane-1,4-diamine]

Dans l'exemple 3, on utilise, comme amine, la N,N'-bis-(3-amino-propyl)-butane-1,4-diamine.
Le produit obtenu est caractérisé spécifiquement par CCM (alcool isopropylique/ammoniaque 28 % / $H_2O$: 6/3/1) : Rf = 0,37.
On a aussi effectué une spectroscopie de masse (electrospray) : $MH^+$ : 603,5.

Exemple 8 : Préparation du Cholest-7-ene-3β, 5α diol 6β-N-[2 éthylamino-(1*H*-imidazol-4-yl)]

Dans l'exemple 3, on utilise, comme amine, la 2-(1*H*-imidazol-4-yl)- éthylamine.
Le produit obtenu est caractérisé spécifiquement par CCM (MeOH) : Rf= 0,38.
On a aussi effectué une spectroscopie de masse (electrospray) : $MH^+$ : 512,5 ; m/z : 365,3.

Exemple 9 : Préparation du Cholestan-3β, 5α diol 6β-N-[2 éthylamino-(1*H*-imidazol-4-yl)]

Dans l'exemple 3, on utilise comme amine la 2-(1*H*-imidazol-4-yl)-éthylamine .
Le produit obtenu est caractérisé spécifiquement par CCM (MeOH) : Rf = 0,38.
On a aussi effectué une chromatographie à haute performance (HPLC) sur un appareil Perkin-Elmer 200 équipé d'une colonne "Ultrasep ES100RP18" (particules de 6 μm), de 250 mm de longueur et 8 mm de diamètre, fabriquée par la société "Bishoff".
HPLCprofil : 44 % B λ = 220 nm tr = 44-50 min
On a enfin effectué une spectroscopie de masse (electrospray) : m/z : 514,5 ($MH^+$).

Exemple 10 : Préparation du Cholest-7-ene-3β, 5α diol 6β-N-(4-amino-butylamine)

Dans l'exemple 3, on utilise, comme amine, la 4-amino-butylamine.
Le produit obtenu est caractérisé spécifiquement par CCM (MeOH/ammoniaque 28 % : 8/2) : Rf= 0,55.
On a aussi effectué une spectroscopie de masse (electrospray) : m/z : 489 ($MH^+$).

Exemple 11 : Préparation du Cholest-7-ene-3β, 5α diol 6β-N-(2-[2-(2-amino-éthoxy)-éthoxy]-éthylamine)

Dans l'exemple 3, on utilise, comme amine, la 2-{ 2-[2-(2-amino-éthoxy)-éthoxy]-éthoxy}-éthylamine.
Le produit obtenu est caractérisé spécifiquement par CCM (MeOH/ammoniaque 28 % / 8/2) : Rf= 0,8.
On a aussi effectué une spectroscopie de masse (electrospray) : m/z : 549 ($MH^+$).

Exemple 12 : Préparation du Cholestan-3β, 5α diol 6β-N-[4-(2-amino-éthyl)-imidazol-1-yl]

Dans l'exemple 3, on utilise, comme amine, la 2-(3H-imidazol-4-yl) éthylamine.
Le produit obtenu est caractérisé spécifiquement par CCM (MeOH/ammoniaque 28 %, 8/2) : Rf = 0,72.
On a aussi effectué une HPLC sur un appareil Perkin-Elmer 200 équipé d'une colonne "Ultrasep ES100RP18" (particules de 6 μm), de 250 mm de longueur et 8 mm de diamètre, fabriquée par la société "Bishoff".
HPLC profil : 44 %B λ = 220 nm tr = 44-50 min
On a enfin effectué une spectroscopie de masse (electrospray) : m/z : 514,5 ($MH^+$).

Exemple 13 : Préparation du Cholestan-3β, 5α diol 6β-N-({1H-imidazol-4-yl}-éthyl)-acétamide

Dans l'exemple 3, on utilise, comme amine, le N-[2-(1H-imidazol-4-yl)-éthyl]-acétamide.
Le produit obtenu est caractérisé spécifiquement par CCM (MeOH/acétate d'éthyle, 1/1) : Rf = 0,5.
On a aussi effectué une HPLC sur un appareil Perkin-Elmer 200 équipé d'une colonne colonne "Ultrasep ES100RP18" (particules de 6 μm), de 250 mm de longueur et 8 mm de diamètre, fabriquée par la société "Bishoff".
HPLC profil : 44 %B λ = 220 nm tr = 44-50 min
On a enfin effectué une spectroscopie de masse (electrospray) : m/z : 557 ($MH^+$).

Exemple 14 : Préparation du Cholestan-3β, 5α diol 6β-(3-amino-propylamine)

Dans l'exemple 3, on utilise, comme amine, le 1,3-diaminopropane.
Le produit obtenu est caractérisé spécifiquement par CCM (MeOH/ammoniaque 28 % : 8/2) : Rf = 0,50.
On a aussi effectué une spectroscopie de masse (électrospray) m/z : 447 ($MH^+$).

Exemple 15 : Préparation du Cholestan-3β, 5α diol 6β-(3-amino-propyl-acétamide)

Dans l'exemple 3, on utilise, comme amine, le N-(3-amino-propyl)-acétamine.

Le produit obtenu est caractérisé spécifiquement par CCM (MeOH/acétate d'éthyle : 8/2) : Rf = 0,54.

On a aussi effectué une spectroscopie de masse (électrospray) m/z: 519 ($MH^+$).

Exemple 16 : Préparation du Cholestan-3β, 5α diol 6β-(4-amino-butylamine)

Dans l'exemple 3, on utilise, comme amine, le 1,4-diaminobutane.

Le produit obtenu est caractérisé spécifiquement par CCM (MeOH/ammoniaque 28 % : 8/2) : Rf = 0,51.

On a aussi effectué une spectroscopie de masse (électrospray) m/z : 491 ($MH^+$).

Exemple 17 : Préparation du Cholestan-3β, 5α diol 6β-(4-amino-butylyl-1-acétamide)

Dans l'exemple 3, on utilise, comme amine, le N-(4-amino-butyl)-acétamide.

Le produit obtenu est caractérisé spécifiquement par CCM (MeOH/acétate d'éthyle : 8/2) : Rf = 0,56.

On a aussi effectué une spectroscopie de masse (électrospray) m/z : 533 ($MH^+$).

Exemple 18 : Préparation du Cholestan-3β, 5α diol 6β-(6-amino-hexylamine)

Dans l'exemple 3, on utilise, comme amine, le 1,6-diaminohexane.

Le produit obtenu est caractérisé spécifiquement par CCM (MeOH/ammoniaque 28 % : 8/2) : Rf = 0,52.

On a aussi effectué une spectroscopie de masse (électrospray) m/z : 519 ($MH^+$).

Exemple 19 : Préparation du Cholestan-3β, 5α diol 6β-(6-amino-hexyl-acétamide)

Dans l'exemple 3, on utilise, comme amine, le N-(6-amino-hexyl)-acétamide.

Le produit obtenu est caractérisé spécifiquement par CCM (MeOH/acétate d'éthyle : 28 % : 8/2) : Rf = 0,57.

On a aussi effectué une spectroscopie de masse (électrospray) m/z : 561 ($MH^+$).

Exemple 20 : Préparation du Cholestene-7-3β, 5α diol 6β-(3-amino-propylamine)

Dans l'exemple 3, on utilise, comme amine, le 1,3-diaminopropane.

Le produit obtenu est caractérisé spécifiquement par CCM (MeOH/ammoniaque 28 % : 8/2) : Rf = 0,50.

On a aussi effectué une spectroscopie de masse (électrospray) m/z : 475 ($MH^+$).

Exemple 21 : Préparation du Cholestene-7-3β, 5α diol 6β-(3-amino-propyl-acétamide)

Dans l'exemple 3, on utilise, comme amine, le N-(3-amino-propyl)-acétamide.

Le produit obtenu est caractérisé spécifiquement par CCM (MeOH/acétate d'éthyle : 8/2) : Rf= 0,54.

On a aussi effectué une spectroscopie de masse (électrospray) m/z : 517 ($MH^+$).

Exemple 22 : Préparation du Cholest-7-ene-3β, 5α diol 6β-(4-amino-butylyl-1-acétamide)

Dans l'exemple 3, on utilise, comme amine, le N-(4-amino-butylacétamide.

Le produit obtenu est caractérisé spécifiquement par CCM (MeOH/acétate d'éthyle : 8/2) : Rf = 0,56.

On a aussi effectué une spectroscopie de masse (électrospray) m/z : 531 ($MH^+$).

Exemple 23 : Préparation du Cholestene-7-3β, 5α diol 6β-(6-amino-hexylamine)

Dans l'exemple 3, on utilise, comme amine, le 1,6-diaminohexane.

Le produit obtenu est caractérisé spécifiquement par CCM (MeOH/ammoniaque 28 % : 8/2) : Rf = 0,51.

On a aussi effectué une spectroscopie de masse (électrospray) m/z : 517 ($MH^+$).

Exemple 24 : Préparation du Cholestene-7-3β, 5α diol 6β-(6-amino-hexyl-acétamide)

Dans l'exemple 3, on utilise, comme amine, le N-(6-amino-hexyl)-acétamide.

Le produit obtenu est caractérisé spécifiquement par CCM (MeOH/acétate d'éthyle : 8/2) : Rf= 0,57.

On a aussi effectué une spectroscopie de masse (électrospray) m/z : 559 ($MH^+$).

Exemple 25 : Préparation du Cholestan-3β, 5α diol 6β-{[2-(1H-imidazol-4-yl)-éthyl]-méthyl-amine}

Dans l'exemple 3, on utilise, comme amine, la [2-(1H-imidazol-4-yl)-éthyl]-méthyl-amine.
Le produit obtenu est caractérisé spécifiquement par CCM (MeOH/ammoniaque 28 % : 8/2) : Rf = 0,51.
On a aussi effectué une spectroscopie de masse (électrospray) m/z : 528 ($MH^+$).

Exemple 26 : Préparation du Cholest-7-ene-3β, 5α diol 6β-{[2-(1H-imidazol-4-yl)-éthyl]-méthyl-amine}

Dans l'exemple 3, on utilise, comme amine, la [2-(1H-imidazol-4-yl)-éthyl]-méthyl-amine.
Le produit obtenu est caractérisé spécifiquement par CCM (MeOH/ammoniaque 28 % : 8/2) : Rf = 0,51.
On a aussi effectué une spectroscopie de masse (électrospray) m/z : 526 ($MH^+$).

Exemple 27 : Préparation du Cholest-7-ene-3β, 5α diol 6β-{[2-(1H-imidazol-4-yl)-éthyl]-méthyl-amine}

Dans l'exemple 3, on utilise, comme amine, la [2-(1H-imidazol-4-yl)-éthyl]-méthyl-amine.
Le produit obtenu est caractérisé spécifiquement par CCM (MeOH/ammoniaque 28 % : 8/2) : Rf = 0,51.
On a aussi effectué une spectroscopie de masse (électrospray) m/z : 526 ($MH^+$).

Exemple 28 : Préparation du 5,6-α-époxy-β-sitostan-3β-ol

Dans l'exemple 1, on utilise, comme stérol, le β-sitostérol.
Le produit obtenu est caractérisé spécifiquement par CCM (acétate d'éthyle) : Rf = 0,69.
Analyse élémentaire : théorique : C = 80,87 ; H = 11,70 ; O = 7,43 trouvé : C = 80.88 ; H = 11.69 ; O = 7.42.
Spectroscopie de masse : DCI/$NH_3$ $MH^+$432.

Exemple 29 : Préparation du 5,6-α-époxy-campestan-3β-ol

Dans l'exemple 1, on utilise, comme stérol, le campestérol.
Le produit obtenu est caractérisé spécifiquement par CCM (acétate d'éthyle) : Rf = 0,69.
Analyse élémentaire : théorique : C = 80,71 ; H = 11,61 ; O = 7,68 trouvé : C = 80.66 ; H = 11.63 ; O = 7.72.
Spectroscopie de masse : DCI/$NH_3$ $MH^+$ 418.

Exemple 30 : Préparation du Sitostan-3β,5α diol 6β-(3-amino-propylamine)

Dans l'exemple 3, on utilise, comme amine, le 1,3-diaminopropane et, comme époxyde, l'époxyde de l'exemple 28.
Le produit obtenu est caractérisé spécifiquement par CCM (MeOH/ammoniaque 28 % : 8/2) : Rf = 0,50.
On a aussi effectué une spectroscopie de masse (électrospray) m/z :505 ($MH^+$).

Exemple 31 : Préparation du Sitostan-3β, 5α diol 6β-(3-amino-propyl-acétamide)

Dans l'exemple 3, on utilise, comme amine, le N-(3-amino-propyl)-acétamide et, comme époxyde, l'époxyde de l'exemple 28.
Le produit obtenu est caractérisé spécifiquement par CCM (MeOH/acétate d'éthyle : 8/2) : Rf= 0,54.
On a aussi effectué une spectroscopie de masse (électrospray) m/z : 547 ($MH^+$).

Exemple 32 : Préparation du Sitostan-3β, 5α diol 6β-(4-amino-butylamine)

Dans l'exemple 3, on utilise, comme amine, le 1,4-diaminobutane et, comme époxyde, l'époxyde de l'exemple 28.
Le produit obtenu est caractérisé spécifiquement par CCM (MeOH/ammoniaque 28 % : 8/2) : Rf = 0,51.
On a aussi effectué une spectroscopie de masse (électrospray) m/z : 519 ($MH^+$).

Exemple 33 : Préparation du Cholestan-3β, 5α diol 6β-(4-amino-butyl-1-acétamide)

Dans l'exemple 3, on utilise, comme amine, le N-(4-amino-butyl)-acétamide et, comme époxyde, l'époxyde de

l'exemple 29.

Le produit obtenu est caractérisé spécifiquement par CCM (MeOH/acétate d'éthyle : 8/2) : Rf = 0,56.

On a aussi effectué une spectroscopie de masse (électrospray) m/z : 561 ($MH^+$).

Exemple 34 : Préparation du Campestan-3β, 5α diol 6β-(3-amino-propylamine)

Dans l'exemple 3, on utilise, comme amine, le 1,3-diaminopropane et, comme époxyde, l'époxyde de l'exemple 29.

Le produit obtenu est caractérisé spécifiquement par CCM (MeOH/ammoniaque 28 % : 8/2) : Rf = 0,50.

On a aussi effectué une spectroscopie de masse (électrospray) m/z : 491 ($MH^+$).

Exemple 35 : Préparation du Campestan-3β, 5α diol 6β-(3-amino-propyl-acétamide)

Dans l'exemple 3, on utilise, comme amine, le N-(3-amino-propyl)-acétamide et, comme époxyde, l'époxyde de l'exemple 29.

Le produit obtenu est caractérisé spécifiquement par CCM (MeOH/acétate d'éthyle : 8/2) : Rf = 0,54.

On a aussi effectué une spectroscopie de masse (électrospray) m/z : 533 ($MH^+$).

Exemple 36 : Préparation du Campestan-3β, 5α diol 6β-(4-amine-butylamine)

Dans l'exemple 3, on utilise, comme amine, le 1,4-diaminobutane et, comme époxyde, l'époxyde de l'exemple 29.

Le produit obtenu est caractérisé spécifiquement par CCM (MeOH/ammoniaque 28 % : 8/2) : Rf = 0,51.

On a aussi effectué une spectroscopie de masse (électrospray) m/z : 505 ($MH^+$).

Exemple 37 : Préparation du Campestan-3β, 5α diol 6β-(4-amine-butyl-1-acétamide)

Dans l'exemple 3, on utilise, comme amine, le N-(4-amino-butyl)-acétamide et, comme époxyde, l'époxyde de l'exemple 29.

Le produit obtenu est caractérisé spécifiquement par CCM (MeOH/acétate d'éthyle : 8/2) : Rf = 0,56.

On a aussi effectué une spectroscopie de masse (électrospray) m/z : 547 ($MH^+$).

Exemple 38 : Préparation du Sitostan-3β, 5α diol 6β-(6-amine-hexylamine)

Dans l'exemple 3, on utilise, comme amine, le 1,6-diaminohexane et, comme époxyde, l'époxyde de l'exemple 28.

Le produit obtenu est caractérisé spécifiquement par CCM (MeOH/ammoniaque 28 % : 8/2) : Rf= 0,53.

On a aussi effectué une spectroscopie de masse (électrospray) m/z : 547 ($MH^+$).

Exemple 39 : Préparation du Sitostan-3β, 5α diol 6β-(6-amino-hexyl-acétamide)

Dans l'exemple 3, on utilise, comme amine, le N-(6-amino-hexyl)-acétamide et, comme époxyde, l'époxyde de l'exemple 28.

Le produit obtenu est caractérisé spécifiquement par CCM (MeOH/acétate d'éthyle : 8/2) : Rf = 0,58.

On a aussi effectué une spectroscopie de masse (électrospray) m/z : 589 ($MH^+$).

Exemple 40 : Préparation du Campestan-3β, 5α diol 6β-(6-amino-hexylamine)

Dans l'exemple 3, on utilise, comme amine, le 1,6-diaminohexane et, comme époxyde, l'époxyde de l'exemple 29.

Le produit obtenu est caractérisé spécifiquement par CCM (MeOH/ammoniaque 28 % : 8/2) : Rf = 0,52.

On a aussi effectué une spectroscopie de masse (électrospray) m/z : 505 ($MH^+$).

Exemple 41 : Préparation du Campestan-3β, 5α diol 6β-(6-amino-hexyl-acétamide)

Dans l'exemple 3, on utilise, comme amine, le N-(6-amino-hexyl)-acétamide et, comme époxyde, l'époxyde de l'exemple 29.

Le produit obtenu est caractérisé spécifiquement par CCM (MeOH/acétate d'éthyle : 8/2) : Rf = 0,57.

On a aussi effectué une spectroscopie de masse (électrospray) m/z : 547 ($MH^+$).

Exemple 42 : Préparation du Sitostan-3β, 5α diol 6β-N-[*N,N'*-bis-(amino-propyl)-butane-1,4-diamine]

Dans l'exemple 3, on utilise, comme amine, la *N,N'*-bis-(3-amino-propyl)-butane-1,4-diamine et, comme époxyde, l'époxyde de l'exemple 28.
Le produit obtenu est caractérisé spécifiquement par CCM (alcool isopropylique/ammoniaque 28 %/$H_2O$ : 6/3/1) : Rf = 0,39.
On a aussi effectué une spectroscopie de masse (électrospray) m/z : 633.5 ($MH^+$).

Exemple 43 : Préparation du Campestan-3β, 5α diol 6β-N-[*N,N'*-bis-(amino-propyl)-butane-1,4-diamine]

Dans l'exemple 3, on utilise, comme amine, la *N,N'*-bis-(3-amino-propyl)-butane-1,4-diamine et, comme époxyde, l'époxyde de l'exemple 29.
Le produit obtenu est caractérisé spécifiquement par CCM (alcool isopropylique/ammoniaque 28 %/$H_2O$ : 6/3/1) : Rf = 0,38.
On a aussi effectué une spectroscopie de masse (électrospray) m/z : 619.5 ($MH^+$).

Exemple 44 : Préparation du Sitostan-3β, 5α diol 6β-N-[1-*N*1-(3-amino-propyl)-butane-1,4-diamine]

Dans l'exemple 3, on utilise, comme amine, la *N*1-(3-amino-propyl)-butane-1,4-diamine et, comme époxyde, l'époxyde de l'exemple 28.
Le produit obtenu est caractérisé spécifiquement par CCM (alcool isopropylique/ammoniaque 28 %/$H_2O$ : 6/3/1) : Rf = 0,62.
On a aussi effectué une spectroscopie de masse (électrospray) m/z : 576 ($MH^+$).

Exemple 45 : Préparation du Campestan-3β, 5α diol 6β-N-[1-*N*1-(3-amino-propyl)-butane-1,4-diamine]

Dans l'exemple 3, on utilise, comme amine, la *N*1-(3-amino-propyl)-butane-1,4-diamine et, comme époxyde, l'époxyde de l'exemple 29.
Le produit obtenu est caractérisé spécifiquement par CCM (alcool isopropylique/ammoniaque 28 %/$H_2O$ 6/3/1) : Rf = 0,62.
On a aussi effectué une spectroscopie de masse (électrospray) m/z : 562 ($MH^+$).

Exemple 46 : Préparation du Sitostan-3β, 5α diol 6β-N-{[2-(1*H* imidazol-4-yl)-éthyl]-éthyl-amine}

Dans l'exemple 3, on utilise, comme amine, la [2-(1*H*-imidazol-4-yl)-éthyl]-éthyl-amine et, comme époxyde, l'époxyde de l'exemple 28.
Le produit obtenu est caractérisé spécifiquement par CCM (MeOH) : Rf = 0,39.
On a aussi effectué une spectroscopie de masse (électrospray) m/z : 542.5 ($MH^+$).

Exemple 47 : Préparation du Campestan-3β, 5α diol 6β-N-{[2-(1*H*-imidazol-4-yl)-éthyl]-éthyl-amine}

Dans l'exemple 3, on utilise, comme amine, la [2-(1*H*-imidazol-4-yl)-éthyl]-éthyl-amine et, comme époxyde, l'époxyde de l'exemple 29.
Le produit obtenu est caractérisé spécifiquement par CCM (MeOH) : Rf= 0,39.
On a aussi effectué une spectroscopie de masse (électrospray) m/z : 528.5 ($MH^+$).
Les exemples 48 et suivants sont relatifs à la mise en oeuvre des composés selon l'invention.

Exemple 48 : dendritogenèses

1) Sur des monocytes de rate de souris C57BL/6.

Des souris de 5 semaines sont sacrifiées par dislocation cervicale. Elles sont ensuite disséquées. Les rates sont prélevées dans des conditions stériles, puis déposées dans une solution froide (4°C) contenant du milieu de culture sans sérum additionné d'antibiotiques (streptomycine, pénicilline). Les rates sont broyées sous une hotte stérile, puis filtrées sur un filtre de 100 μm. L'éluat est récupéré puis centrifugé à 1000 tours/mn durant 5 minutes à 4°C. Le culot cellulaire est resuspendu dans une solution tampon phosphate salin (TPS) contenant de la collagénase qui a pour but

de lyser les hématies. La suspension est centrifugée à 1000 tours/mn puis les cellules sont resuspendues dans du TPS. L'opération de lavage est effectuée deux fois. La dernière resuspension est réalisée dans du milieu de culture complet, les cellules sont comptées et sont réparties dans des boîtes de six puits à une densité de 40 000 cellules/ml. Quatre heures après, les cellules adhérentes sont lavées vigoureusement par du TPS afin d'éliminer les cellules qui auraient pu rester accrochées aux cellules adhérentes ou à la boîte de culture.

La figure 1 montre les importantes modifications morphologiques sur les monocytes de souris obtenus comme ci-dessus indiqué, la transformation aboutissant à des cellules dendritiques. La transformation a été obtenue avec une dose de 1nM, la photographie ayant été prise 36 heures après le début d'action du produit. Le produit testé est celui de l'exemple 9. On observe l'apparition de prolongements cellulaires, qui sont l'une des principales caractéristiques de cellules dendritiques activées.

Dans le même temps de traitement des cellules, on a constaté que l'IL-4 et le GM-CSF sont inefficaces ; on a constaté également que sont inefficaces les composés, qui ont servi à la synthèse du composé de formule (I) testé, à savoir le cholestérol, le dérivé époxy-cholestérol et l'amine.

Des résultats analogues ont été obtenus avec les composés des exemples 8, 10, 11, 46 ou 47.

2) Sur des cellules leucémiques myéloïdes humaines U-937

La figure 3 représente l'induction de la différentiation des cellules U-937 en cellules dendritiques au moyen du composé de l'exemple 9.

La photographie 1 représente les cellules après un traitement par 10 ng/ml de phorbol-myristyl-acétate (PMA) de manière à les faire adhérer sur le fond des boîtes de culture. La photographie 2 permet, après 2 jours de traitement avec 1 nM du composé de l'exemple 9, d'observer l'apparition de cellules dendritiques (CD). La photographie 3 montre, après 5 jours de traitement, l'augmentation de la taille des dendrites (jusqu'à 100 $\mu$m) et la formation d'un grand nombre de rosettes (R) constituées d'un conglomérat de cellules entrelacées les unes avec les autres.

Les mêmes observations sont effectuées quand on remplace le composé de l'exemple 9 par le composé des exemples 46 ou 47.

<u>Exemple 49</u> : neuritogenèses

1) Sur des cellules PC 12

Les cellules PC12 ont été cultivées à une densité initiale de 1.5 $10^6$ cellules/ml dans du milieu RPMI 1640 complémenté par 10 % de sérum de veau foetal et 5 % de sérum de cheval. Le fond des boîtes de culture est traité par une solution de poly-lysine à 0,1 % en poids ou de collagène afin de provoquer l'adhérence des cellules. Les cellules surnageantes sont éliminées après 12 heures puis traitées avec ou sans Nerve growth factor (NGF) à 10 ng/ml, avec ou sans le composé de l'exemple 6 ou de l'exemple 7. Les cellules sont observées par microscopie de contraste de phase.

Les cellules PC12 "contrôle" ont été traitées avec le véhicule-solvant utilisé pour les composés testés à savoir (eau + 0,1 % en poids d'éthanol) : ces cellules "contrôle" apparaissent rondes et n'évoluent pas sur le plan morphologique au cours du temps.

Les cellules PC 12 traitées au moyen du composé de l'exemple 6 ont été traitées à une dose de 10 nM, le temps d'action du composé étant de 36 heures.

Les cellules PC12 traitées par le composé de l'exemple 6 changent rapidement de morphologie ; elles deviennent ovoïdes après 2 à 4 heures de traitement ; puis on observe l'apparition d'excroissances qui évoluent progressivement vers la formation de dendrites (voir figure 2). On constate que ces cellules PC 12 sont bi-polarisées.

Après 4 heures de traitement des cellules PC 12 avec le composé de l'exemple 6, on observe l'apparition de foyers d'adhérence de cellules dans les boîtes de culture. Ces foyers sont 4 à 6 fois plus nombreux que dans le cas de cellules traitées avec du NGF utilisé à des doses de 10 ng/ml. Le composé testé provoque la pousse de dendrites sur les PC 12 dans des conditions similaires à celles précédemment indiquées pour le traitement avec le composé de l'exemple 6. Les mêmes observations sont effectuées quand on remplace le composé de l'exemple 6 par le composé de l'un des exemples 4, 5, 7, 42, 43, 44 et 45.

2) Sur des cellules P19 de souris

Les cellules P19 dérivent d'un carcinome embryonnaire de souris. Ce sont des cellules pluri-potentes, qui peuvent évoluer en neurones ou en cellules gliales par traitement à l'acide rétinoïque (J. Cell. Biol. 1982 Août, 94(2) : p. 253-262) et peuvent se différencier en cellules musculaires en présence de diméthylsulfoxide (Nature 1982 - 9 septembre, 299 (58 79) : p.165-167).

Les cellules P19 sont cultivées en présence d'un milieu de culture "RPMI 1640" commercialisé par la société

"Gibco BRL", ce milieu étant supplémenté par 2mM de glutamine et 4 % en poids de sérum de veau foetal. Elles sont ensemencées à faible densité (10.000 cellules par puits de 60 mm). Les cellules P19 sont traitées avec le composé de l'exemple 6 ; elles sont ensuite observées en microscopie de contraste de phase. Ces cellules ont été traitées à une dose de 10 nM, le temps d'action du composé étant de 36 heures.

Les cellules P19 traitées avec le composé de l'exemple 6 changent de morphologie après 12 heures de traitement : on observe l'apparition de prolongements cellulaires qui évoluent vers la formation de dendrites (voir figure 16 où la photographie 1 correspond à un contrôle témoin et la photographie 2 correspond aux composés testés.

Les mêmes observations sont effectuées quand on remplace le composé de l'exemple 6 par le composé de l'un des exemples 4, 5, 7, 42, 43, 44 et 45.

Exemple 50 : apparition d'antigènes de surface caractéristique de l'état de différentiation des CD.

L'étude a été menée sur des CD provenant de splenocytes de souris C57BL/6, de PBMC humains et de cellules leucémiques myéloïdes U937. Les cellules provenant de splenocytes de souris C57BL/6 ont été préparées comme indiqué à l'exemple-48.1.

Les phénotypes sont analysés par cytométrie de flux sur un "FAC Scan flow" (Beckton Dikinson, BD Bioscience, Franlin Lakes, NJ, USA) en utilisant des anticorps conjugués au FITC (HLA-DR, CD-80, LAMP-II) et des anticorps conjugués à la Phycoerythrine (anti-CD-83, CD-86 et CD-40 provenant tous de "BD Bioscience").

Pour le composé de l'exemple 9, les résultats sont fournis dans le tableau ci-dessous :

Phénotype des cellules dendritiques stimulées par le composé de l'exemple 9

| Phénotype | CD dérivées de monocytes de souris C57BL/6 | CD dérivées de cellules U-937 | CD dérivées de monocytes humains |
|---|---|---|---|
| | | | |
| CMH de Classe I | +++ | +++ | +++ |
| CMH de Classe II | +++ | +++ | +++ |
| CD 80 | +++ | +++ | +++ |
| CD 40 | +++ | +++ | +++ |
| CD 86 | + | + | +++ |
| LAMP-II | + | + | + |
| CD 83 | ND | + | + |
| ND = non déterminé | | | |

Exemple 51 : production d'IL-12p70 et d'IL-10 par les cellules dendritiques

Les surnageants de cultures sont congelés à une température de -80°C jusqu'à ce qu'ils soient testés pour la présence des cytokines. La présence de IL-12p70 et IL-10 est mesurée à l'aide de kits ELISA obtenus par "Endogen" (Woburn, MA, USA).

La figure 4 représente la production de cytokines par les cellules U-937 traitées par le composé de l'exemple 9. On constate que le traitement stimule la production de la sous-unité p70 de l'IL-12 dès 36 heures après le début du traitement. Ce phénomène se poursuit pendant les deux semaines de traitement. Ce même traitement ne stimule pas la transcription d'IL-10 pour des traitements qui s'étendent sur une durée de deux semaines : ce résultat est particulièrement avantageux dès lors que l'on sait que IL-10 a des propriétés immuno-suppressives, qui bloquent la différentiation des cellules dendritiques et que le couple (IL-4 + GM-CSF) stimule la production d'IL-10.

Les mêmes observations ont été effectuées quand on remplace le composé de l'exemple 9 par le composé de l'un des exemples 46 et 47.

Exemple 52 : prolifération des lymphocytes T

Des monocytes de souris C57BL/6 ont été prélevés comme indiqué à l'exemple 48.1. Les cellules dendritiques

ont été obtenues comme indiqué à l'exemple 9. Après transformation en CD par le composé testé, les cellules sont traitées par un antimitotique (mitomycin C à 0,0006 mg/ml) et incubées pendant 30 mn. à 37°C, afin de bloquer la synthèse d'ADN dans les CD. Après ce traitement, deux lavages des cellules sont réalisés avec du milieu RPMI 1640 complémenté avec 10 % de sérum de veau foetal.

Des rates de souris BALB/c ont également été prélevées et traitées comme cela a été décrit pour la préparation des monocytes de souris C57BL/6. Les cellules non-adhérentes, qui contiennent les lymphocytes T, sont récupérées et ajoutées aux cellules dendritiques de souris C57BL/6. Au quatrième jour de mise en présence des cellules, on ajoute de la thymidine tritiée. Après 18 heures d'incubation, les cellules sont lysées avec de l'alcool absolu. Le précipité est suspendu dans de l'eau puis filtré sur un filtre de cellulose. L'incorporation de thymidine est mesurée par comptage à scintillation (Pakard Instrument, Meriden CT, USA).

La figure 5 représente l'activation des lymphocytes T par les cellules dendritiques produites sous l'effet du composé de l'exemple 9. Les monocytes adhérents provenant de rates de souris obtenues selon l'exemple 14.1 ont été traités par le composé susmentionné à des doses variables. Les cellules dendritiques ainsi obtenues ont été mises en présence des lymphocytes T provenant de rates de souris BALB/c. La prolifération des lymphocytes est mesurée par la mesure de l'incorporation de thymidine tritiée dans l'ADN.

On constate que le composé testé induit une stimulation de la prolifération des lymphocytes T d'un facteur 5. Si l'on effectue le traitement des mêmes cellules par le couple (IL-4/GM-CSF) suivi d'un traitement par le TNF$\alpha$, l'effet de prolifération n'est observé qu'à un niveau beaucoup plus faible puisque cet effet correspond à un facteur 1,4.

Les mêmes observations ont été effectuées quand on remplace le composé de l'exemple 9 par le composé de l'un des exemples 46 et 47.

Exemple 53 : prolifération de cellules d'adénocarcinome mammaire de souris (TS/A (H-$2^d$)) en culture

La lignée tumorale de souris TS/A (H-$2^d$) est une lignée cellulaire d'un adénocarcinome mammaire spontané de souris syngénique des souris BALB/c. Elles sont cultivées dans du milieu RPMI 1640 contrôlé sans micoplasme supplémenté par du sérum de veau foetal contrôlé sans endotoxine (GIRCO-BRIO, 2 mM de glutamine, 100 U/ml de pénicilline, 50 $\mu$g/ml de streptomycine, des acides aminés essentiels et du pyruvate de sodium.

La figure 6 montre la cytotoxicité sur TS/A in vitro : les cellules tumorales ont été traitées par des doses variables du composé de l'exemple 9. La cytotoxicité apparaît pour des doses de 50 $\mu$M. Les doses choisies pour effectuer les tests sur les tumeurs implantées sur les souris BALB/C étaient des doses de 5 nM et 1 $\mu$M pour lesquelles aucune toxicité n'est observée quel que soit le temps d'incubation avec les cellules.

Exemple 54 : Tests de croissance sur tumeurs implantées sur les souris BALB/c

Les tumeurs sont implantées de la façon suivante : $1.10^6$ cellules de TS/A sont inoculées par injection intradermique dans le flanc droit de l'abdomen de souris BALB/c âgées de 5-6 semaines (Elevage Janvier, Le Genest Saint Isle).

La molécule testée est celle qui correspond à l'exemple 9 ; elle est injectée au troisième jour en intradermique à proximité de la tumeur ; le résultat est représenté sur la figure 7.

En l'absence du composé, les tumeurs apparaissent dès quatre jours et poussent durant une période de trente jours. Il apparaît, par rapport aux souris témoins, un effet de ralentissement pour une dose de composé testé de 8,5 ng/g de souris. A la dose de 1,7 $\mu$g/g de souris, aucune tumeur n'est détectable sur les souris. Ceci illustre l'extrême efficacité du traitement. Les analyses d'anatomo-pathologie montrent qu'aucune trace de nécrose n'est observable au niveau de la zone d'injection du composé testé. Pour les souris traitées par le composé testé, à une dose de 1,7 $\mu$g/g, qu'il y ait eu ou non une implantation de tumeur, on observe une hyperplasie du système lymphoïde périphérique significatif d'une activation du système immunitaire.

On a reproduit la même expérience que celle ci-dessus décrit en modifiant la zone d'injection de la molécule correspondant à l'exemple 9 ; l'injection est effectuée au troisième jour après l'implantation de la tumeur, en intradermique, en position contra-latérale par rapport à ladite implantation. Dans cette expérimentation, il apparaît par rapport aux souris témoins un ralentissement de la croissance tumorale pour la dose de 1,7$\mu$g/g de souris. Ceci illustre l'efficacité du traitement pour une administration du composé testé à distance de la tumeur. Les résultats obtenus sont représentés par les courbes de la figure 17.

Les mêmes observations ont été effectuées quand on remplace le composé de l'exemple 9 par le composé de l'un des exemples 46 et 47.

Exemple 55 : Induction de la production de vacuoles dans la lignée cellulaire tumorale A 549

Des cellules A549 ont été traitées avec 100 nM de composé selon l'exemple 9 pendant 12 heures.

Les cellules A549 ont été obtenues de l'American Tissue Culture Collection (ATCC) ; elles ont été ensemencées à une densité de 50.000 cellules par puits dans des plaques de 12 puits et ont été cultivées dans du milieu RPMI supplémenté avec 5 % de sérum de veau foetal. Pour effectuer le traitement avec le composé selon l'invention, les cellules sont incubées avec ledit composé (ou avec un produit contrôle) pendant 48 heures. Les cellules sont alors observées en microscopie de contraste de phase toutes les quatre heures pour surveiller la formation de vacuoles puis elles sont fixées avec une solution de 0,4 % de paraformaldéhyde dans du tampon phosphate salin (TPS) à pH 7.4.

Le résultat est représenté sur la figure 8 (microscopie en contraste de phase). La photographie A montre les cellules avant traitement et la photographie B après traitement ; sur la photographie C, les cellules ont été traitées avec le véhicule solvant ; et sur la photographie D, on constate que le traitement avec le composé selon l'invention conduit à une apparition massive de vacuoles.

Pour obtenir la photographie D ci-dessus indiquée, on a procédé comme suit : après le traitement des cellules, le milieu de culture est retiré et les cellules sont lavées à l'aide de TPS à pH 7.4. Les cellules sont incubées pendant 10 mn par des solutions 1 μM de monodansyl cadavérine (MDC). Elles sont ensuite rincées abondamment par une solution de TPS à 4°C. Une goutte de solution de "Vectashield" (Vector laboratoires, CA) est ajoutée avant le montage entre lame et lamelle : les cellules sont ensuite observées rapidement au microscope et photographiées. La photographie D de la figure 8 montre de nombreuses vacuoles fluorescentes dans les cellules.

On constate donc que le composé testé induit une production massive de vacuoles cytoplasmiques dans la lignée tumorale humaine A549. L'effet constaté est dose-dépendant et il apparaît six heures après le traitement ; il atteint un maximum douze heures après le traitement.

Les mêmes observations ont été effectuées quand on remplace le composé de l'exemple 9 par le composé de l'un des exemples 46 et 47.

On a constaté que le composé de l'exemple 8 montrait également un effet analogue. En outre, un phénomène analogue a été observé sur les cellules U-937, PC-12, MCF-7, HELA, COS-7, HEK-293, HEK-293T, NIH-3T3, HT-29 et CHO obtenues et traitées comme indiqué ci-dessus pour les cellules A 549.

Lorsque le traitement des cellules est stoppé en remplaçant le milieu de croissance par un milieu frais, les vacuoles disparaissent : ceci montre que l'effet du composé selon l'invention est réversible.

Les cellules A549 traitées comme indiqué ci-dessus avec le composé selon l'exemple 9 ont aussi été examinées en microscopie électronique. Pour examiner ces A549 en microscopie électronique, on les fixe à l'aide d'une solution de TPS contenant 3 % de glutaraldéhyde, on les prend dans de la résine (EPON) et enfin on les coupe en fines lamelles à l'ultra-microtome. Ces lamelles sont alors déposées sur des grilles en cuivre de 200 mesh puis examinées en microscopie électronique. Les résultats sont présentés sur la figure 11. Sur la figure 11.1, on voit les cellules "contrôle" (grossissement 6.000) ; sur la figure 11.2 on voit les cellules traitées par le composé de l'exemple 9 (grossissement 4.000) ; sur la figure 11.3, on voit un agrandissement des vésicules détectables sur la figure 11.2 (grossissement 25.000) ; sur la figure 11.4, on voit un détail du contenu d'une vacuole intracytoplasmique (grossissement 72.000). Sur cette figure 11, les lettres utilisées ont les significations suivantes : N = noyau ; C= cytoplasme ; M = mitochondries ; Vi = vésicules intracytoplasmiques ; Ve = vésicules extracellulaires ; CML = corps multilamellaire.

L'analyse détaillée des photographies de la figure 11 permet les observations suivantes : on observe sur la figure 11.1 une cellule ayant un noyau volumineux centré, entouré par le cytoplasme ; à la surface de la cellule, on observe de petits prolongements, qui sont des villi. Sur la photographie 11.2, on voit la cellule après qu'elle ait subi un traitement par le composé selon l'invention ; elle a adopté une morphologie fusiforme ; les villi sont concentrés sur une face de la cellule (dans le quart droit en bas sur la figure 11.2) où apparaissent en surface de nombreuses vacuoles ; on observe également de nombreuses vacuoles et mitochondries dans le cytoplasme. Sur la photographie 11.3, on observe de nombreuses mitochondries ainsi que des vacuoles, qui contiennent des corps sombres et qui sont entourées de fibres de cytosquelette ; sur cette vue, deux types de vacuoles sont observables : des vacuoles intracytoplasmiques (Vi) et des vacuoles extracytoplasmiques (Ve). Sur la figure 11.4, on voit un détail d'une structure contenue dans les vacuoles intracytoplasmiques : il s'agit d'un corps multilamellaire caractéristique des cellules pulmonaires produisant des protéines de type surfactant B.

<u>Exemple 56</u> : Survie de cellules PC 12

Des cellules PC 12 ont été traitées comme indiqué à l'exemple 49.1. Dans le cas du présent exemple, elles sont traitées par des doses croissantes d'un composé selon l'invention utilisé à 100 nM ; elles sont maintenues dans des boîtes de culture pendant trois semaines. Le nombre de cellules vivantes est mesuré tous les deux jours. Le composé testé est ajouté tous les deux jours pendant les six premiers jours du traitement.

Ce test a été effectué avec le composé de l'exemple 6 et le composé de l'exemple 7. Les cellules PC12 ont été incubées à 37°C avec chacun des composés testés et avec 10 ng/ml de NGF : ces substances ont été rajoutées au milieu de culture lors de l'ensemencement, les résultats sont exprimés en pourcentages moyens de survie des cellules PC12 pour trois expériences indépendantes : ils sont représentés sur la figure 9.

Les mêmes observations sont effectuées quand on remplace le composé de l'exemple 6 par le composé de l'un des exemples 4, 5, 42, 43, 44 et 45.

Les cellules PC 12 ainsi traitées sont maintenues en culture après avoir subi la pousse dendritique dont l'existence a été établie par l'exemple 15. On constate que la survie de ces cellules est, par rapport à l'utilisation du NGF, considérablement augmentée lorsqu'on utilise les composés selon l'invention.

Exemple 57 : Survie de moto-neurones

On a extrait des neurones de souris et on les a maintenus en culture selon la méthode décrite par Duong et Coll., British Journal of Pharmacology, 1999, vol. 28, p. 1385-1392. Les cellules maintenues en culture ont été traitées par le véhicule-solvant utilisé (eau + diméthyl sulfoxyde 0,02 %), par un facteur neurotrophique (à savoir le BDNF, "Brain Derived Neurotrophic Factor") ou par un composé selon l'invention. On a utilisé des doses variables de composé afin d'évaluer les propriétés des composés selon l'invention sur les moto-neurones normaux. La méthodologie utilisée pour les cultures et le comptage des cellules survivantes est la même que celle définie à l'exemple 56 ; les moto-neurones purifiés ont été incubés à 37°C avec le véhicule-solvant ci-dessus indiqué, avec 10 ng/ml de BDNF ou avec le composé de l'exemple 6 ou de l'exemple 7. à une concentration de 100 nM. Les résultats sont représentés sur la figure 10.

Les mêmes observations sont effectuées quand on remplace le composé de l'exemple 6 par le composé de l'un des exemples 4, 5, 42, 43, 44 et 45.

On constate qu'en l'absence de traitement avec un composé selon l'invention ou avec le BDNF, les cellules disparaissent rapidement au bout de quelques jours. On constate que l'efficacité de l'un ou l'autre des composés selon l'invention qui ont été testés est supérieure à celle du BDNF : l'ajout d'un composé selon l'invention entraîne donc non seulement la pousse dendritique (comme établi par l'exemple 49), mais également la survie des cellules pendant une période de trois semaines.

Les tests de survie donnés dans les exemples 56 et 57 sont indicatifs d'un effet favorable des molécules sur des maladies neurodégénératrices telles que la sclérose latérale amyotrophique (Amyotroph Lateral Scler Other Motor Neuron Discord (2001) 2 mars ; suppl. 1 : S 55-68) ou la maladie d'Alsheimer et la maladie de Parkinson (Brain Research Review (2000) vol. 3, p. 199-227).

Exemple 58 : squelette d'actine sur les cellules A549 traitées

Des cellules A549 ont été traitées par le composé de l'exemple 9 selon le protocole décrit en détail dans l'exemple 55 ; elles sont ensemencées sur des lamelles de verre placées au fond de boîtes de culture à six puits (NUNC) à une densité de 50.000 cellules par puits dans un milieu nutritif de type "RPMI 1640" commercialisé par la société "Gibco BRL", complémenté à 10 % en sérum de veau foetal. Les cellules sont alors fixées par une solution à 3 % de paraformaldéhyde et elles sont perméabilisées par une solution détergente contenant 0,1 % de "Triton-X-100" dans du TPS. On a observé le squelette d'actine des cellules traitées en marquant les cellules avec la phalloïdine-FITC.

Le résultat est présenté sur la figure 12 : la photographie 1 de la figure 12 montre les cellules A549 témoin : le cytosquelette d'actine enserre les cellules de façon régulière. La photographie 2 de la figure 12 montre l'apparition de vésicules réfringentes, qui sont entourées d'actine ; de telles vésicules sont également apparentes à l'extérieur de la cellule : on pense que l'actine intervient comme moteur lors de la sécrétion des vacuoles d'exocytose mises en évidence dans l'exemple 55.

Sur la figure 13, on a présenté l'observation du squelette de tubuline sur les cellules A549 en microscopie de fluorescence. La photographie 1 montre une cellule témoin non traitée et la photographie 2 montre une cellule traitée selon le processus donné en détail dans l'exemple 21. Le marquage des cellules A549 se fait ici avec un anticorps anti-tubuline $\gamma$ avec révélation avec un anti-corps anti-IgG-FITC.

Sur la photographie 1, on observe un réseau filamenteux régulier. Sur la photographie 2, on observe la présence de vacuoles intracytoplasmiques, qui ne sont pas entourées de tubuline $\gamma$ (apparition de nombreux trous qui délimitent la présence de ces vacuoles).

Les observations combinées des figures 12 et 13 montrent que les fibres de cytosquelette observables en microscopie électronique dans les cellules A549 traitées contiennent des fibres d'actine.

Les mêmes observations ont été effectuées quand on remplace le composé de l'exemple 9 par le composé de l'un des exemples 46 et 47.

Exemple 59 : induction de la production de vacuoles dans la lignée cellulaire tumorale U937

Des cellules U937 ont été traitées avec 10 nM du composé selon l'exemple 9 pendant 24 heures. Les modalités du traitement sont celles qui ont été exposées en détail à l'exemple 55.

Le résultat est représenté sur la figure 14. Sur cette figure, la photographie 1 correspond à une cellule "témoin"

(grossissement 6.000). La photographie 2 correspond à une cellule traitée par le composé de l'exemple 9 (grossissement 4.000) : on constate que la cellule a changé de forme et fait apparaître de nombreuses vacuoles intracytoplasmiques (repérées Vi sur la photographie). Les significations des lettres symbole sur la figure 14 sont les mêmes que celles qui ont été utilisées pour la figure 11. Sur la photographie 3 (grossissement 25.000) on constate la présence d'un réseau membranaire extrêmement développé ; on distingue de nombreux organites cellulaires (nombreuses mitochondries témoignant d'une activité intense) ; on distingue également des corps multi-vésiculaires (CMV), dont le détail apparaît sur la photographie 4 de la figure 14, cette photographie repésentant un CMV en cours de sécrétion à l'extérieur de la cellule (grossissement 72.000).

Les mêmes observations ont été effectuées quand on remplace le composé de l'exemple 9 par le composé de l'un des exemples 46 et 47.

<u>Exemple 60</u> : sur-expression d'un récepteur membranaire dans la lignée cellulaire tumorale U937

Des cellules U937 ont été cultivées dans un milieu "RPMI 1640" commercialisé par la société "Gibco BRL". On génère un plasmide codant un récepteur membranaire RCPG-MA qui appartient à la super-famille des récepteurs couplés aux protéines G et qui contient une étiquette HA. Les cellules U937 exprimant les récepteurs sont générées par transfection en utilisant le réactif "lipofectamine" selon les instructions du fabricant.

48 heures après la transfection, on a traité les cellules transfectées par 10 nM de composé selon l'exemple 9 pendant 12 heures.

La figure 15 présente deux photographies d'immunofluorescence de cellules U937 qui expriment le RCPG-HA et qui ont subi un traitement par le composé de l'exemple 9. Ces cellules ont été fixées pour être examinées en microscopie de fluorescence. La présence du récepteur est révélée par incubation en présence d'un anticorps anti-HA suivie d'une incubation avec un anticorps anti-IgG-FITC. Les photographies de la figure 15 montrent l'expression d'un récepteur membranaire de type RCPG dans les cellules qui ont été traitées par le composé selon l'invention, alors qu'en l'absence de traitement l'immuno-détection est difficilement réalisée. Les mêmes observations sont effectuées quand on remplace le composé de l'exemple 9 par le composé de l'un des exemples 10, 11, 14, 16, 46 et 47.

Cette expérience montre que les cellules transfectées peuvent produire massivement un récepteur membranaire dans les vacuoles intracytoplasmiques.

Les exemples 48 et suivants, qui ont été ci-dessus exposés, montrent que les types cellulaires soumis aux expérimentations produisent tous des vacuoles lorsqu'ils sont traités avec l'un des composés selon l'invention : selon les cas, il peut s'agir de vacuoles multi-lamellaires et/ou multi-vésiculaires. On constate que le traitement par un composé selon l'invention permet de constater dans les cellules traitées une activité de re-différentiation des cellules tumorales vers un phénotype normal.

## Revendications

1. Composé dérivé de stérol, **caractérisé par le fait qu'**il correspond à la formule (I)

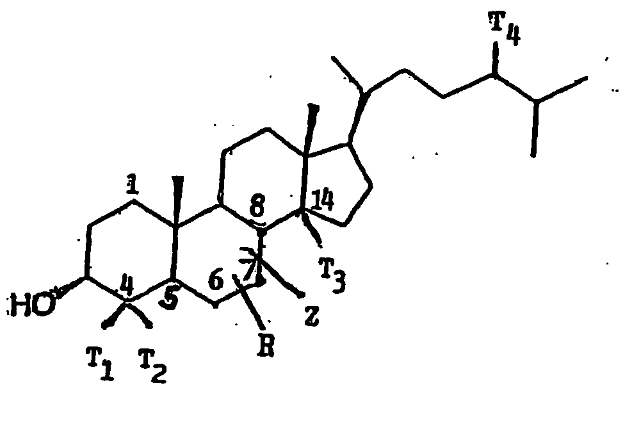

(I)

formule dans laquelle le carbone en position 4 du squelette cholestérol porte des éléments $T_1$, $T_2$, qui peuvent être indépendamment H ou $CH_3$ avec $CH_3$ en $\alpha$ et/ou $\beta$, le carbone en position 24 porte un élément $T_4$ qui représente H, $CH_3$ ou $C_2H_5$, le carbone en position 14 porte un élément $T_3$, qui peut être H ou un $CH_3$ en alpha, l'une des liaisons entre les carbones 5 et 6, d'une part, et 7 et 8, d'autre part, peut être double alors que l'autre est simple,

et dans laquelle :

- Z représente, en position 5 ou 8, soit H soit OH, OH ne pouvant être porté que par un carbone qui ne porte pas de double liaison ;
- et R représente en position 6 ou 7, sur un carbone ne portant pas de double liaison, le substituant de formule $-Q_0-Q_1$,

substituant dans la formule duquel
-Qo- représente le radical de formule (II) :

$$-X-(CH_2)_{n0}\ [Y_1-(CH_2)_{n1}]_{p1}\ [Y_2-\ (CH_2)_{n2}\ ]_{p2}\ (Y_3-(CH_2)_{n3}\ ]_{p3}\ [Y_4-(CH_2)_{n4}\ ]_{p4}\ [Y_5-\ (CH_2)_{n5}\ ]_{p5}- \quad (II)$$

formule (II) dans laquelle :

. $p_1$, $p_2$, $p_3$, $p_4$, $p_5$ sont des nombres entiers égaux, indépendamment les uns des autres, à 0 ou 1,
. $n_0$, $n_1$, $n_2$, $n_3$, $n_4$, $n_5$ sont des nombres entiers indépendants les uns des autres tels que :

$$1 \leq n_0 \leq 4$$

$$0 \leq n_1, n_2, n_3, n_4, n_5 \leq 4$$

. -X- représente -S-, -O-, $-CH_2-$, $-NR_3-$, où $R_3$ est H ou un radical alkyl en $C_1$-$C_4$, ou encore un hétérocycle

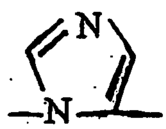

. $-Y_1$, $-Y_2-$, $-Y_3-$, $-Y_4-$, $-Y_5-$ représentent, indépendamment les uns des autres, -S-, -O-, -C-, $-NR_3-$, où $R_3$ a la signification ci-dessus indiquée ;

et dans la formule duquel

- $Q_1$ représente un noyau indolique, un noyau morpholinique ou thiomorpholinique rattaché par son atome d'azote, un hétérocycle

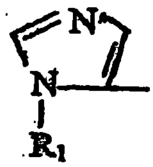

où $R_1$ représente H, $COCH_3$, un radical alkyl en $C_1$-$C_4$ , ou encore

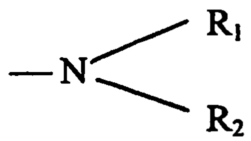

où $R_1$ a les significations ci-dessus indiquées et $R_2$ représente H ou un radical alkyl en $C_1$-$C_4$, $R_1$ et $R_2$ pouvant

constituer ensemble un cycle pipéridinique, pyridinique, pipérazinique éventuellement substitué par un radical alkyl en $C_1$-$C_4$, ou encore un hétérocycle pyrolique ou pyrolidinique comportant un atome d'azote et 4 atomes de carbone, sous réserve que :

. si -X- = -NH- et

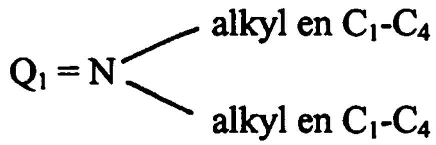

,

l'un au moins des nombres $p_1$, $p_2$, $p_3$, $p_4$, $p_5$ soit différent de 0 ;
• si -X- = -$CH_2$-, n0 = 1 et tous les nombres $p_1$, $p_2$, $p_3$, $p_4$, $p_5$ sont nuls, $Q_1$ soit différent de -$NH_2$.

2. Composé selon la revendication 1, **caractérisé par le fait qu'**il correspond à la formule (I) dans laquelle la liaison entre les carbones $C_7$ et $C_8$ est double, R = NH-$(CH_2)_3$-NH-$(CH_2)_4$-$NH_2$ et $T_1 = T_2 = T_3$ = H.

3. Composé selon la revendication 1, **caractérisé par le fait qu'**il correspond à la formule (I) dans laquelle la liaison entre les carbones $C_7$ et $C_8$ est double, $T_1 = T_2 = T_3$ = H et
R = -NH-$(CH_2)_3$-NH-$(CH_2)_4$-NH-$(CH_2)_3$-$NH_2$.

4. Composé selon la revendication 1, **caractérisé par le fait qu'**il correspond à la formule (I) dans laquelle la liaison entre les carbones $C_7$ et $C_8$ est double, $T_1 = T_2 = T_3$ = H et

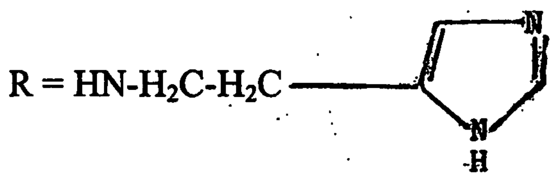

5. Composé selon la revendication 1, **caractérisé par le fait qu'**il correspond à la formule (I) dans laquelle la liaison entre les carbones $C_7$ et $C_8$ est double, $T_1 = T_2 = T_3$ = H et R = -NH-$(CH_2)_4$-$NH_2$.

6. Composé selon la revendication 1, **caractérisé par le fait qu'**il correspond à la formule (I) dans laquelle la liaison $C_7$-$C_8$ est double, $T_1 = T_2 = T_3$ = H et R = -NH-$(CH_2)_2$-O-$(CH_2)_2$-O-$(CH_2)_2$-$NH_2$.

7. Composé selon la revendication 1, **caractérisé par le fait qu'**il correspond à la formule (I) dans laquelle les deux liaisons $C_5$-$C_6$ et $C_7$-$C_8$ sont simples, Z représente OH en position 5, $T_1 = T_2 = T_3$ = H, R étant en position 6 et ayant la même signification que dans la revendication 3.

8. Composé selon la revendication 1, **caractérisé par le fait qu'**il correspond à la formule (I) dans laquelle les deux liaisons $C_5$-$C_6$ et $C_7$-$C_8$ sont simples, Z représente OH en position 5, $T_1 = T_2 = T_3$ = H, R étant en position 6 et ayant la même signification que dans la revendication 4.

9. Composé selon la revendication 1, **caractérisé par le fait qu'**il correspond à la formule (I) dans laquelle les deux liaisons $C_5$-$C_6$ et $C_7$-$C_8$ sont simples, Z représente OH en position 5, $T_1 = T_2 = T_3$ = H, R étant en position 6 et ayant la signification

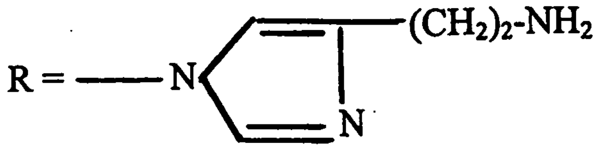

**10.** Composé selon la revendication 1, **caractérisé par le fait qu'**il correspond à la formule (I) dans laquelle les deux liaisons $C_5$-$C_6$ et $C_7$-$C_8$ sont simples, Z représente OH en position 5, $T_1$= $T_2$ = $T_3$ = H, R étant en position 6 et ayant la signification

R = 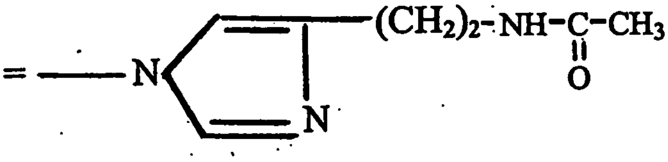

**11.** Composé selon la revendication 1, **caractérisé par le fait qu'**il correspond à la formule (I) dans laquelle les deux liaisons $C_5$-$C_6$ et $C_7$-$C_8$ sont simples, Z représente OH en position 5, $T_1$ = $T_2$ = $T_3$ = H, R étant en position 6 et ayant la même signification que dans la revendication 2.

**12.** Procédé de préparation d'un composé selon la revendication 1, **caractérisé par le fait que** :

- dans une première étape, on fait agir l'acide meta-chloroperoxybenzoïque, dissout dans un solvant A, sur un composé répondant à la formule (III)

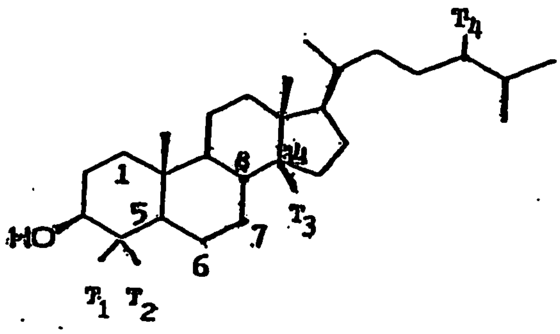

(III)

formule dans laquelle le carbone en position 4 du squelette cholestérol porte des éléments $T_1$, T2, qui peuvent être indépendamment H ou $CH_3$ avec $CH_3$ en $\alpha$ et/ou $\beta$, le carbone en position 24 porte un élément $T_4$ qui représente H, $CH_3$ ou $C_2H_5$, le carbone en position 14 porte un élément $T_3$, qui peut être H ou un $CH_3$ en alpha, l'une au moins des liaisons entre les carbones 5 et 6, d'une part, et 7 et 8, d'autre part, est double, le composé de formule III étant dissous dans un solvant B miscible au solvant A,

- dans une deuxième étape, on fait agir sur le composé époxy obtenu par la première étape, dissous dans un solvant C en présence d'un activateur D, une amine de formule $Q_0Q_1$, $Q_0$ et $Q_1$ ayant les significations indiquées dans la revendication 1, dissoute dans un solvant E miscible au solvant C.

**13.** Procédé selon la revendication 12, **caractérisé par le fait que** l'on purifie le produit obtenu dans la première étape avant de l'utiliser pour la deuxième étape.

**14.** Procédé selon l'une des revendications 12 ou 13, **caractérisé par le fait que** l'on utilise, comme activateur D, le perchlorate de lithium.

**15.** Procédé selon l'une des revendications 12 à 14, **caractérisé par le fait que** l'on utilise, comme solvant A, le chlorure de méthylène.

**16.** Procédé selon la revendication 15, pour la préparation d'un composé de formule (I) portant un OH sur le carbone en position 5 et comportant une double liaison entre les carbones 7 et 8, **caractérisé par le fait que** l'on utilise, comme solvant B, un mélange de chlorure de méthylène et d'une solution aqueuse de $Na_2CO_3$.

**17.** Procédé selon la revendication 15, pour la préparation d'un composé de formule (I) portant un OH sur le carbone en position 5 et comportant une simple liaison entre les carbones 7 et 8, **caractérisé par le fait que** l'on utilise,

comme solvant B, du chlorure de méthylène.

**18.** Procédé selon l'une des revendications 16 ou 17, **caractérisé par le fait que** l'on utilise, comme solvant C, de l'éthanol anhydre ou de la pyridine, la réaction de la deuxième étape étant réalisée au reflux, à la pression atmosphérique.

**19.** Médicament, **caractérisé par le fait qu'**il comporte, dans un véhicule pharmaceutiquement acceptable, au moins un composé selon la revendication 1.

**20.** Médicament selon la revendication 19 pour utilisation pour augmenter la dendritogénèse de cellules vivantes de mammifères.

**21.** Médicament selon la revendication 20, pour utilisation pour déclencher une neuritogénèse sur des cellules nerveuses ou leurs précurseurs.

**22.** Médicament selon la revendication 21, pour utilisation pour combattre les maladies neurodégénératives humaines, notamment la sclérose latérale amiotrophique, la maladie d'Alzheimer et la maladie de Parkinson.

**23.** Médicament selon la revendication 19, pour utilisation pour activer le système immunitaire d'un organisme vivant.

**24.** Médicament selon la revendication 19, pour utilisation pour la production de vacuoles sécrétoires dans des cellules tumorales d'un organisme vivant.

**25.** Médicament selon la revendication 24, pour utilisation pour faire régresser une tumeur cancéreuse de mammifère.

**26.** Médicament selon l'une des revendications 19 à 25, **caractérisé par le fait qu'**il est sous forme injectable.

## Claims

**1.** A sterol-based compound, **characterized in that** it corresponds to formula (I)

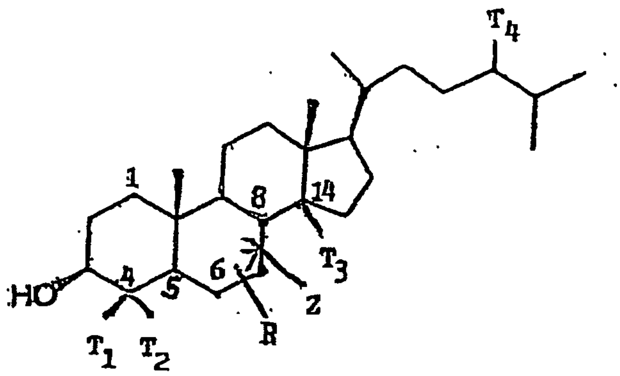

(I)

in which formula the carbon in position 4 of the cholesterol skeleton bears moieties $T_1$ and $T_2$, which may be, independently, H or $CH_3$ with $CH_3$ in the $\alpha$ and/or $\beta$ position, the carbon in position 24 bears a moiety $T_4$ which represents H, $CH_3$ or $C_2H_5$, the carbon in position 14 bears a moiety $T_3$, which may be H or a $\alpha$ $CH_3$, one of the bonds between carbons 5 and 6, on the one hand, and 7 and 8, on the other hand, may be a double bond, whereas the other is a single bond, and in which:

- Z represents, in position 5 or 8, either H or OH, OH being able to be borne only by a carbon that does not bear a double bond; and
- R represents in position 6 or 7, on a carbon not bearing a double bond, the substituent of formula $-Q_0-Q_1$,

in the formula of which substituent

$-Q_0-$ represents the radical of formula (II):

$$- X-(CH_2)_{n0}\ [Y_1-(CH_2)_{n1}]_{p1}\ [Y_2-(CH_2)_{n2}]_{p2}\ [Y_3-(CH_2)_{n3}]_{p3}\ [Y_4-(CH_2)_{n4}]_{p4}\ [Y_5-(CH_2)_{n5}]_{p5}- \quad (II)$$

in which formula (II):

. p1, p2, p3, p4 and p5 are integers independently equal to 0 or 1,
. n0, n1, n2, n3, n4 and n5 are independent integers such that:

$$1 \le n0 \le 4$$

$$0 \le n1, n2, n3, n4, n5 \le 4$$

. -X- represents -S-, -O-, $-CH_2-$ or $-NR_3-$, in which $R_3$ is H or a $C_1$-$C_4$ alkyl radical, or alternatively a heterocycle

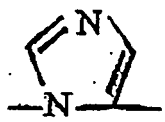

. $-Y_1-$, $-Y_2-$, $-Y_3-$, $-Y_4-$ and $-Y_5-$ represent, independently of each other, -S-, -O-, -C- or $-NR_3-$, in which $R_3$ has the meaning given above;

and in which formula

- $Q_1$ represents an indole nucleus, a morpholine or thiomorpholine nucleus attached via its nitrogen atom, a heterocycle

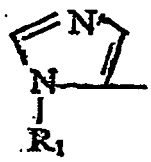

in which $R_1$ represents H, $COCH_3$, a $C_1$-$C_4$ alkyl radical, or

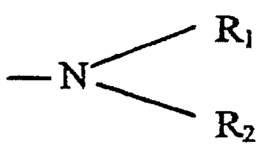

in which $R_1$ has the meanings given above and $R_2$ represents H or a $C_1$-$C_4$ alkyl radical, $R_1$ and $R_2$ together possibly constituting a piperidine, pyridine or piperazine ring optionally substituted with a $C_1$-$C_4$ alkyl radical, or alternatively a pyrrole or pyrrolidine heterocycle comprising a nitrogen atom and 4 carbon atoms, with the proviso that:

. if -X- = -NH- and

$Q_1$ = 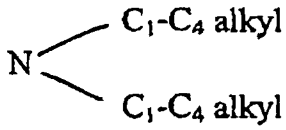 ,

at least one of the numbers p1, p2, p3, p4 and p5 is other than 0; and

. if -X- = $-CH_2-$, n0 = 1 and all the numbers p1, p2, p3, p4 and p5 are zero, $Q_1$ is other than $-NH_2$.

**2.** The compound as claimed in claim 1, **characterized in that** it corresponds to formula (I) in which the bond between carbons $C_7$ and $C_8$ is a double bond, R = $NH-(CH_2)_3-NH-(CH_2)_4-NH_2$ and $T_1 = T_2 = T_3 = H$.

**3.** The compound as claimed in claim 1, **characterized in that** it corresponds to formula (I) in which the bond between carbons $C_7$ and $C_8$ is a double bond, $T_1 = T_2 = T_3 = H$ and R = $-NH-(CH_2)_3-NH-(CH_2)_4-NH-(CH_2)_3-NH_2$.

**4.** The compound as claimed in claim 1, **characterized in that** it corresponds to formula (I) in which the bond between carbons $C_7$ and $C_8$ is a double bond, $T_1$=$T_2$=$T_3$=H and

R = 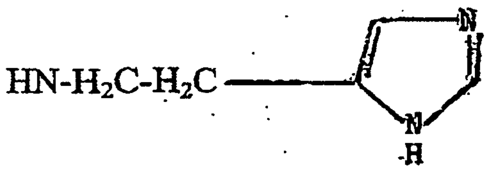

**5.** The compound as claimed in claim 1, **characterized in that** it corresponds to formula (I) in which the bond between carbons $C_7$ and $C_8$ is a double bond, $T_1 = T_2 = T_3 = H$ and R = $-NH-(CH_2)_4-NH_2$.

**6.** The compound as claimed in claim 1, **characterized in that** it corresponds to formula (I) in which the bond $C_7$-$C_8$ is a double bond, $T_1 = T_2 = T_3 = H$ and R = $-NH-(CH_2)_2-O-(CH_2)_2-O-(CH_2)_2-NH_2$.

**7.** The compound as claimed in claim 1, **characterized in that** it corresponds to formula (I) in which the two bonds $C_5$-$C_6$ and $C_7$-$C_8$ are single bonds, Z represents OH in position 5 and $T_1 = T_2 = T_3 = H$, R being in position 6 and having the same meaning as in claim 3.

**8.** The compound as claimed in claim 1, **characterized in that** it corresponds to formula (I) in which the two bonds $C_5$-$C_6$ and $C_7$-$C_8$ are single bonds, Z represents OH in position 5 and $T_1 = T_2 = T_3 = H$, R being in position 6 and having the same meaning as in claim 4.

**9.** The compound as claimed in claim 1, **characterized in that** it corresponds to formula (I) in which the two bonds $C_5$-$C_6$ and $C_7$-$C_8$ are single bonds, Z represents OH in position 5 and $T_1 = T_2 = T_3 = H$, R being in position 6 and having the meaning

R = 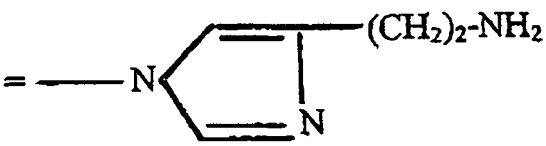

**10.** The compound as claimed in claim 1, **characterized in that** it corresponds to formula (I) in which the two bonds $C_5$-$C_6$ and $C_7$-$C_8$ are single bonds, Z represents OH in position 5 and $T_1 = T_2 = T_3 = H$, R being in position 6 and having the meaning

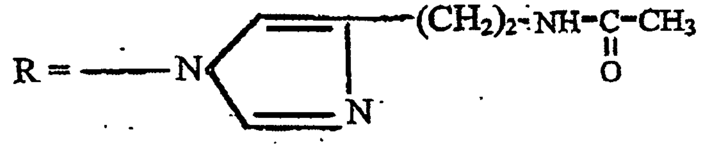

**11.** The compound as claimed in claim 1, **characterized in that** it corresponds to formula (I) in which the two bonds $C_5$-$C_6$ and $C_7$-$C_8$ are single bonds, Z represents OH in position 5 and $T_1 = T_2 = T_3 = H$, R being in position 6 and having the same meaning as in claim 2.

**12.** A process for preparing a compound as claimed in claim 1, **characterized in that**:

- in a first step, meta-chloroperoxybenzoic acid, dissolved in a solvent A, is reacted with a compound corresponding to formula (III)

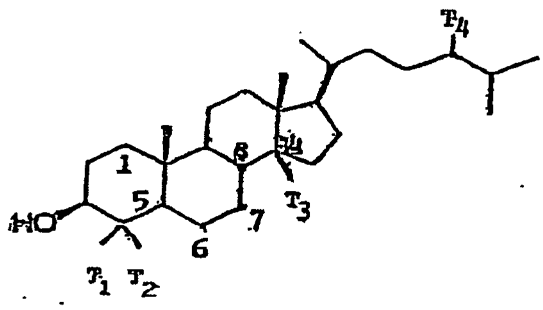

(III)

in which formula the carbon in position 4 of the cholesterol skeleton bears moieties $T_1$ and $T_2$ which may be, independently, H or $CH_3$ with $CH_3$ in the $\alpha$ and/or $\beta$ position, the carbon in position 24 bears a moiety $T_4$ that represents H, $CH_3$ or $C_2H_5$, the carbon in position 14 bears a moiety, $T_3$, which may be H or a $\alpha$ $CH_3$, at least one of the bonds between carbons 5 and 6, on the one hand, and 7 and 8, on the other hand, is a double bond, the compound of formula III being dissolved in a solvent B that is miscible with solvent A,
- in a second step, the epoxy compound obtained in the first step, dissolved in a solvent C in the presence of an activator D, is reacted with an amine of formula $Q_0Q_1$, $Q_0$ and $Q_1$ having the meanings given in claim 1, dissolved in a solvent E that is miscible with the solvent C.

**13.** The process as claimed in claim 12, **characterized in that** the product obtained in the first step is purified before using it for the second step.

**14.** The process as claimed in either of claims 12 and 13, **characterized in that** lithium perchlorate is used as activator D.

**15.** The process as claimed in one of claims 12 to 14, **characterized in that** methylene chloride is used as solvent A.

**16.** The process as claimed in claim 15, for the preparation of a compound of formula (I) bearing an OH on the carbon in position 5 and comprising a double bond between carbons 7 and 8, **characterized in that** a mixture of methylene chloride and of aqueous $Na_2CO_3$ solution is used as solvent B.

**17.** The process as claimed in claim 15, for the preparation of a compound of formula (I) bearing an OH on the carbon in position 5 and comprising a single bond between carbons 7 and 8, **characterized in that** methylene chloride is used as solvent B.

**18.** The process as claimed in either of claims 16 and 17, **characterized in that** anhydrous ethanol or pyridine is used as solvent C, the reaction of the second step being performed at reflux, at atmospheric pressure.

**19.** A medicament, **characterized in that** it comprises, in a pharmaceutically acceptable vehicle, at least one compound as claimed in claim 1.

**20.** Medicament as claimed in claim 19, for use to increase the dendritogenesis of live mammalian cells.

**21.** Medicament as claimed in claim 20, for use to trigger neuritogenesis on nerve cells or precursors thereof.

**22.** Medicament as claimed in claim 21, **characterized in that** it is used to combat human neurodegenerative diseases, especially amyotrophic lateral sclerosis, Alzheimer's disease and Parkinson's disease.

**23.** Medicament as claimed in claim 19, for use to activate the immune system of a live organism.

**24.** Medicament as claimed in claim 19, for use for the production of secretory vacuoles in tumoral cells of a live organism.

**25.** Medicament as claimed in claim 24, for use to regress a mammalian cancer tumor.

**26.** Medicament as claimed in one of claims 19 to 25, **characterized in that** it is under injectable form.

**Patentansprüche**

**1.** Sterinderivat-Verbindung, **dadurch gekennzeichnet, dass** diese der Formel (I) entspricht,

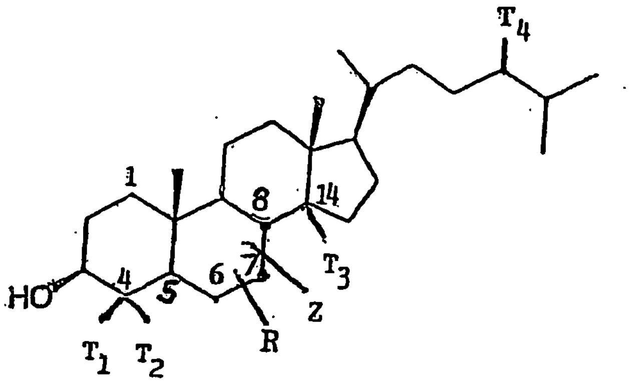

(I)

wobei in der Formel der Kohlenstoff in Position 4 des Cholesteringerüsts Elemente $T_1$, $T_2$ trägt, die unabhängig voneinander für H oder $CH_3$, mit $CH_3$ in $\alpha$ und/oder $\beta$, stehen können, der Kohlenstoff in Position 24 ein Element $T_4$ trägt, das für H, $CH_3$ oder $C_2H_5$ steht, der Kohlenstoff in Position 14 ein Element $T_3$ trägt, das für H oder ein $CH_3$ in $\alpha$ stehen kann, eine der Bindungen zwischen den Kohlenstoffen 5 und 6 einerseits, und 7 und 8 andererseits, eine Doppelbindung sein kann, während die andere eine Einfachbindung ist und wobei:

- Z in Position 5 oder 8, entweder für H oder OH steht, wobei OH nur durch ein Kohlenstoffatom, das keine Doppelbindung trägt, getragen werden kann;
- und R in Position 6 oder 7, an einem Kohlenstoffatom, das keine Doppelbindung trägt, für den Substituenten der Formel $-Q_0-Q_1$ steht,

wobei in der Formel des Substituenten
$-Q_0-$ für einen Rest der Formel (II) steht:

$$- X-(CH_2)_{no}\ [Y_1-(CH_2)_{n1}]_{p1}\ [Y_2-(CH_2)_{n2}]_{p2}\ [Y_3-(CH_2)_{n3}]_{p3}\ [Y_4-(CH_2)_{n4}]_{p4}\ [Y_5-(CH_2)_{n5}]_{p5}-$$
(II)

wobei in der Formel (II):

■ $p_1$, $p_2$, $p_3$, $p_4$, $p_5$ unabhängig voneinander jeweils für ganze Zahlen von 0 oder 1 stehen,
■ $n_0$, $n_1$, $n_2$, $n_3$, $n_4$, $n_5$ unabhängig voneinander für ganze Zahlen stehen, dass:

$$1 \leq n_0 \leq 4$$

$$0 \leq n_1, n_2, n_3, n_4, n_5 \leq 4$$

■ -X- für -S-, -O-, $-CH_2-$, $-NR_3-$ steht, worin $R_3$ für H oder einen $C_1$-$C_4$-Alkylrest oder einen Heterocyclus

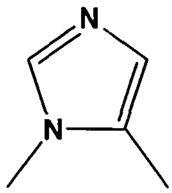

steht,
■ $-Y_1-$, $-Y_2-$, $-Y_3-$, $-Y_4-$, $-Y_5-$ unabhängig voneinander für -S-, -O-, -C-, $-NR_3-$ stehen, worin $R_3$ die oben gegebene Bedeutung aufweist;

und wobei in dessen Formel

- $Q_1$ für einen Indolkern, einen Morpholin- oder Thiomorpholinkern, gebunden über sein Stickstoffatom, einen Heterocyclus,

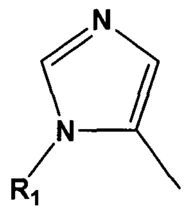

worin $R_1$ für H, $COCH_3$, einen $C_1$-$C_4$-Alkylrest, oder aber

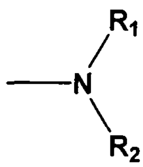

steht,
worin $R_1$ die oben gegebenen Bedeutungen aufweist und $R_2$ für H oder einen $C_1$-$C_4$-Alkylrest steht, wobei $R_1$ und $R_2$ zusammen einen Piperidin-, Pyridin-, Piperazinring, gegebenenfalls substituiert durch einen $C_1$-$C_4$-Alkylrest, oder einen Pyrrol-oder Pyrrolidinheterocyclus, umfassend ein Stickstoffatom und 4 Kohlenstoffatome, bilden können, unter der Voraussetzung, dass:

- wenn -X- = -NH- und

$Q_1$ = 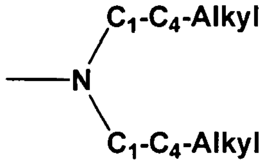 ,

wenigstens einer der Werte $p_1$, $p_2$, $p_3$, $p_4$, $p_5$ verschieden von 0 ist;
- wenn -X- = $-CH_2-$, $n_0$ = 1 und alle Werte $p_1$, $p_2$, $p_3$, $p_4$, $p_5$ Null sind, $Q_1$ verschieden von $-NH_2$ ist.

**2.** Verbindung gemäß Anspruch 1, **dadurch gekennzeichnet, dass** diese der Formel (I) entspricht, worin die Bindung zwischen dem $C_7$- und dem $C_8$-Kohlenstoffatom eine Doppelbindung ist, R = $NH-(CH_2)_3-NH-(CH_2)_4-NH_2$ und $T_1 = T_2 = T_3 = H$.

**3.** Verbindung gemäß Anspruch 1, **dadurch gekennzeichnet, dass** diese der Formel (I) entspricht, worin die Bindung zwischen dem $C_7$- und dem $C_8$-Kohlenstoffatom eine Doppelbindung ist, $T_1 = T_2 = T_3 = H$ und R = $-NH-(CH_2)_3-NH-(CH_2)_4-NH-(CH_2)_3-NH_2$.

**4.** Verbindung gemäß Anspruch 1, **dadurch gekennzeichnet, dass** diese der Formel (I) entspricht, worin die Bindung zwischen dem $C_7$- und dem $C_8$-Kohlenstoffatom eine Doppelbindung ist, $T_1 = T_2 = T_3 = H$ und

R = 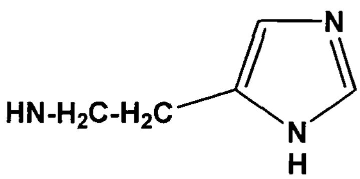 .

**5.** Verbindung gemäß Anspruch 1, **dadurch gekennzeichnet, dass** diese der Formel (I) entspricht, worin die Bindung zwischen dem $C_7$- und dem $C_8$-Kohlenstoffatom eine Doppelbindung ist, $T_1 = T_2 = T_3 = H$ und R = $-NH-(CH_2)_4-NH_2$.

**6.** Verbindung gemäß Anspruch 1, **dadurch gekennzeichnet, dass** diese der Formel (I) entspricht, worin die Bindung zwischen dem $C_7$- und dem $C_8$-Kohlenstoffatom eine Doppelbindung ist, $T_1 = T_2 = T_3 = H$ und R = $-NH-(CH_2)_2-O-(CH_2)_2-O-(CH_2)_2-NH_2$.

**7.** Verbindung gemäß Anspruch 1, **dadurch gekennzeichnet, dass** diese der Formel (I) entspricht, worin die beiden Bindungen $C_5$-$C_6$ und $C_7$-$C_8$ Einfachbindungen sind, Z für OH in Position 5 steht, $T_1 = T_2 = T_3 = H$, wobei sich R in Position 6 befindet und dieselbe Bedeutung wie in Anspruch 3 aufweist.

**8.** Verbindung gemäß Anspruch 1, **dadurch gekennzeichnet, dass** diese der Formel (I) entspricht, worin die beiden Bindungen $C_5$-$C_6$ und $C_7$-$C_8$ Einfachbindungen sind, Z für OH in Position 5 steht, $T_1 = T_2 = T_3 = H$, wobei sich R in Position 6 befindet und dieselbe Bedeutung wie in Anspruch 4 aufweist.

**9.** Verbindung gemäß Anspruch 1, **dadurch gekennzeichnet, dass** diese der Formel (I) entspricht, worin die beiden Bindungen $C_5$-$C_6$ und $C_7$-$C_8$ Einfachbindungen sind, Z für OH in Position 5 steht, $T_1 = T_2 = T_3 = H$, wobei sich R in Position 6 befindet und folgende Bedeutung aufweist

R = 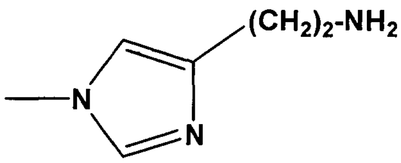 .

**10.** Verbindung gemäß Anspruch 1, **dadurch gekennzeichnet, dass** diese der Formel (I) entspricht, worin die beiden

Bindungen $C_5$-$C_6$ und $C_7$-$C_8$ Einfachbindungen sind, Z für OH in Position 5 steht, $T_1 = T_2 = T_3 = H$, wobei sich R in Position 6 befindet und folgende Bedeutung aufweist

R = 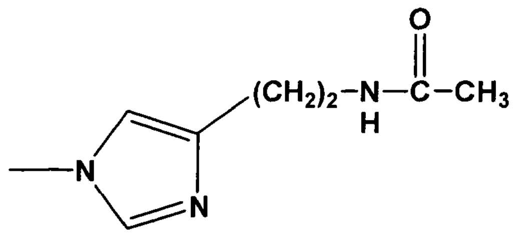

**11.** Verbindung gemäß Anspruch 1, **dadurch gekennzeichnet, dass** diese der Formel (I) entspricht, worin die beiden Bindungen $C_5$-$C_6$ und $C_7$-$C_8$ Einfachbindungen sind, Z für OH in Position 5 steht, $T_1 = T_2 = T_3 = H$, wobei sich R in Position 6 befindet und dieselbe Bedeutung wie in Anspruch 2 aufweist.

**12.** Verfahren zur Herstellung einer Verbindung gemäß Anspruch 1, **dadurch gekennzeichnet, dass** man:

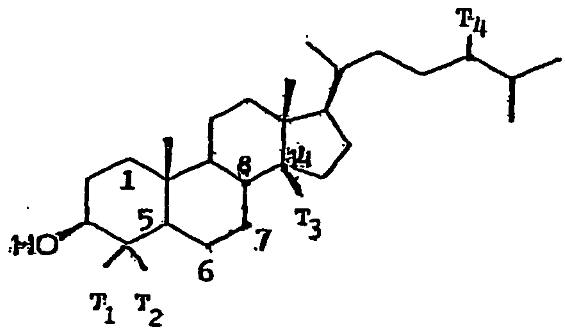

(III)

- in einer ersten Stufe meta-Chlorperbenzoesäure, gelöst in einem Lösungsmittel A, mit einer Verbindung der Formel (III) umsetzt,

wobei in der Formel der Kohlenstoff in Position 4 des Cholesteringerüsts Elemente $T_1$, $T_2$ trägt, die unabhängig voneinander für H oder $CH_3$, mit $CH_3$ in $\alpha$ und/oder $\beta$, stehen, der Kohlenstoff in Position 24 ein Element $T_4$ trägt, das für H, $CH_3$ oder $C_2H_5$ steht, der Kohlenstoff in Position 14 ein Element $T_3$ trägt, das für H oder $CH_3$ in $\alpha$ stehen kann, wobei wenigstens eine der Bindungen zwischen den Kohlenstoffen 5 und 6 einerseits und 7 und 8 andererseits eine Doppelbindung ist, wobei die Verbindung der Formel III in einem mit dem Lösungsmittel A mischbaren Lösungsmittel B gelöst ist,

- in einer zweiten Stufe die in der ersten Stufe erhaltene Epoxyverbindung, gelöst in einem Lösungsmittel C, in Gegenwart eines Aktivators D mit einem Amin der Formel $Q_0Q_1$, worin $Q_0$ und $Q_1$ die in Anspruch 1 gegebenen Bedeutungen aufweisen, gelöst in einem mit dem Lösungsmittel C mischbaren Lösungsmittel E, umsetzt.

**13.** Verfahren gemäß Anspruch 12, **dadurch gekennzeichnet, dass** man das in der ersten Stufe erhaltene Produkt reinigt, bevor man es für die zweite Stufe verwendet.

**14.** Verfahren gemäß einem der Ansprüche 12 oder 13, **dadurch gekennzeichnet, dass** man als Aktivator D Lithiumperchlorat verwendet.

**15.** Verfahren gemäß einem der Ansprüche 12 bis 14, **dadurch gekennzeichnet, dass** man als Lösungsmittel A Methylenchlorid verwendet.

**16.** Verfahren gemäß Anspruch 15, zur Herstellung einer Verbindung der Formel (I), die ein OH am Kohlenstoff in Position 5 trägt und eine Doppelbindung zwischen den Kohlenstoffen 7 und 8 umfasst, **dadurch gekennzeichnet, dass** man als Lösungsmittel B eine Mischung von Methylenchlorid und einer wässrigen Lösung von $Na_2CO_3$ verwendet.

**17.** Verfahren gemäß Anspruch 15, zur Herstellung einer Verbindung der Formel (I), die ein OH am Kohlenstoff in Position 5 trägt und eine Einfachbindung zwischen den Kohlenstoffen 7 und 8 umfasst, **dadurch gekennzeichnet, dass** man als Lösungsmittel B Methylenchlorid verwendet.

**18.** Verfahren gemäß einem der Ansprüche 16 oder 17, **dadurch gekennzeichnet, dass** man als Lösungsmittel C wasserfreies Ethanol oder Pyridin verwendet, wobei die Reaktion der zweiten Stufe unter Rückfluss bei Atmosphärendruck durchgeführt wird.

**19.** Medikament, **dadurch gekennzeichnet, dass** es in einem pharmazeutisch verträglichen Träger wenigstens eine Verbindung gemäß Anspruch 1 umfasst.

**20.** Medikament gemäß Anspruch 19, für die Verwendung zur Erhöhung der Dendritogenese von lebenden Zellen von Säugetieren.

**21.** Medikament gemäß Anspruch 20, für die Verwendung zum Auslösen einer Neuritogenese an Nervenzellen oder deren Vorläufern.

**22.** Medikament gemäß Anspruch 21, für die Verwendung zur Bekämpfung von menschlichen neurodegenerativen Krankheiten, insbesondere der amyotrophen Lateralsklerose, der Alzheimer-Krankheit und der Parkinson-Krankheit.

**23.** Medikament gemäß Anspruch 19, für die Verwendung zur Aktivierung des Immunsystems eines lebenden Organismus.

**24.** Medikament gemäß Anspruch 19, für die Verwendung zur Bildung von sekretorischen Vakuolen in tumorösen Zellen eines lebenden Organismus.

**25.** Medikament gemäß Anspruch 24, für die Verwendung zum Bewirken eines Rückgangs eines krebsartigen Tumors von Säugetieren.

**26.** Medikament gemäß den Ansprüchen 19 bis 25, **dadurch gekennzeichnet, dass** es eine injizierbare Form aufweist.

FIG. 1

Contrôle

Composé testé

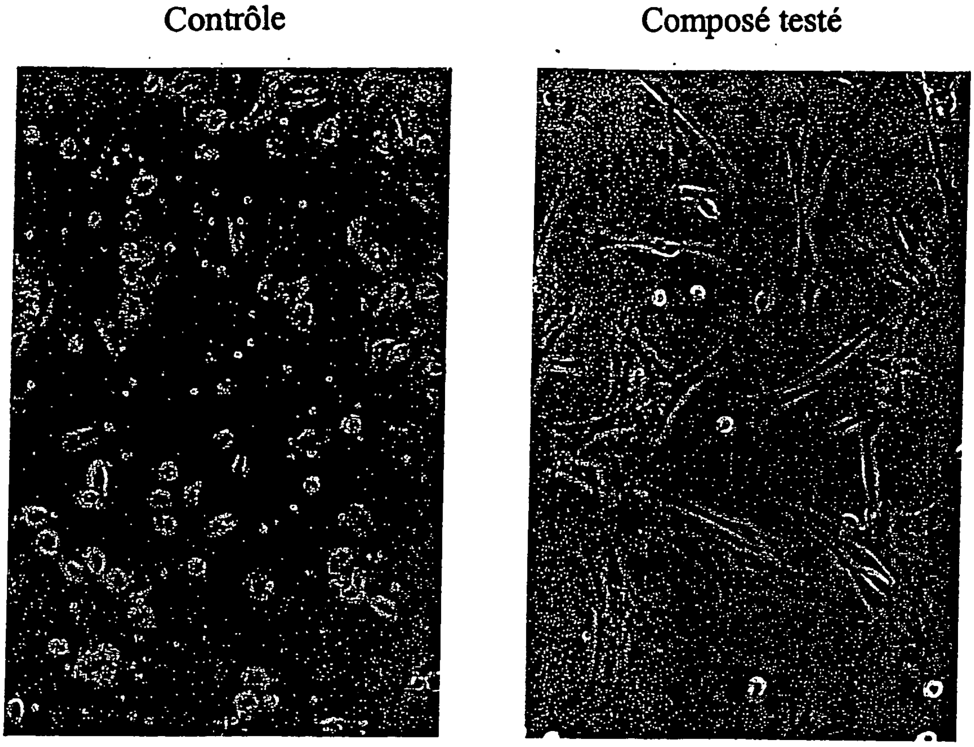

FIG. 2

Contrôle

Composé testé

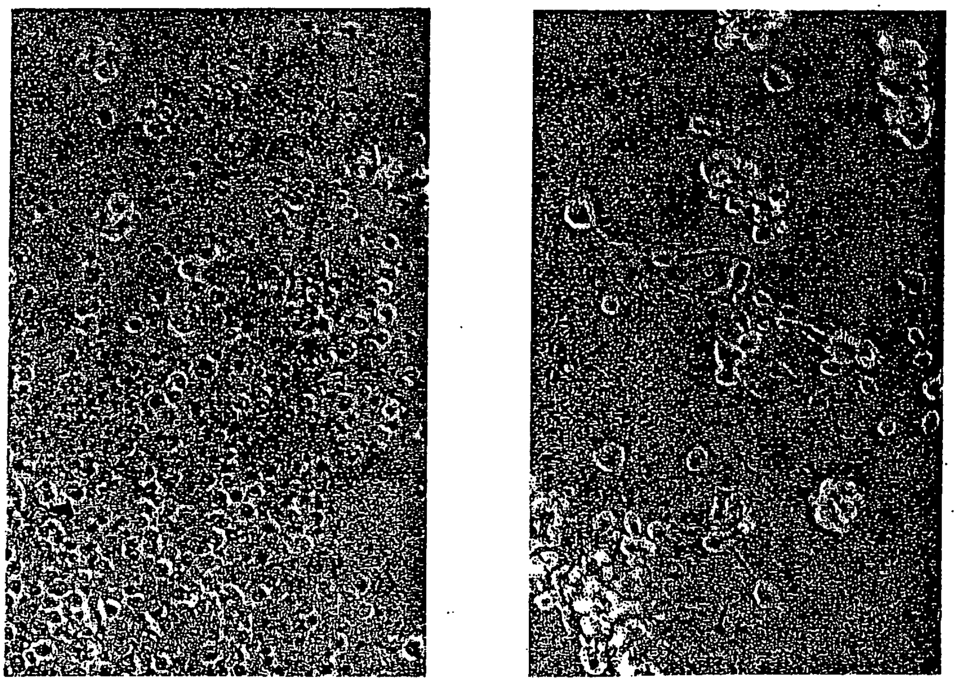

FIG. 3

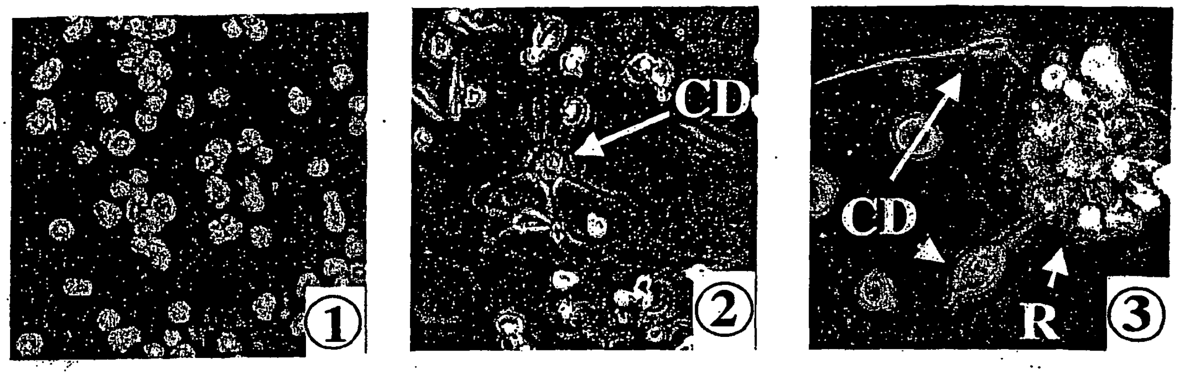
CD
CD
R
①
②
③

FIG. 4

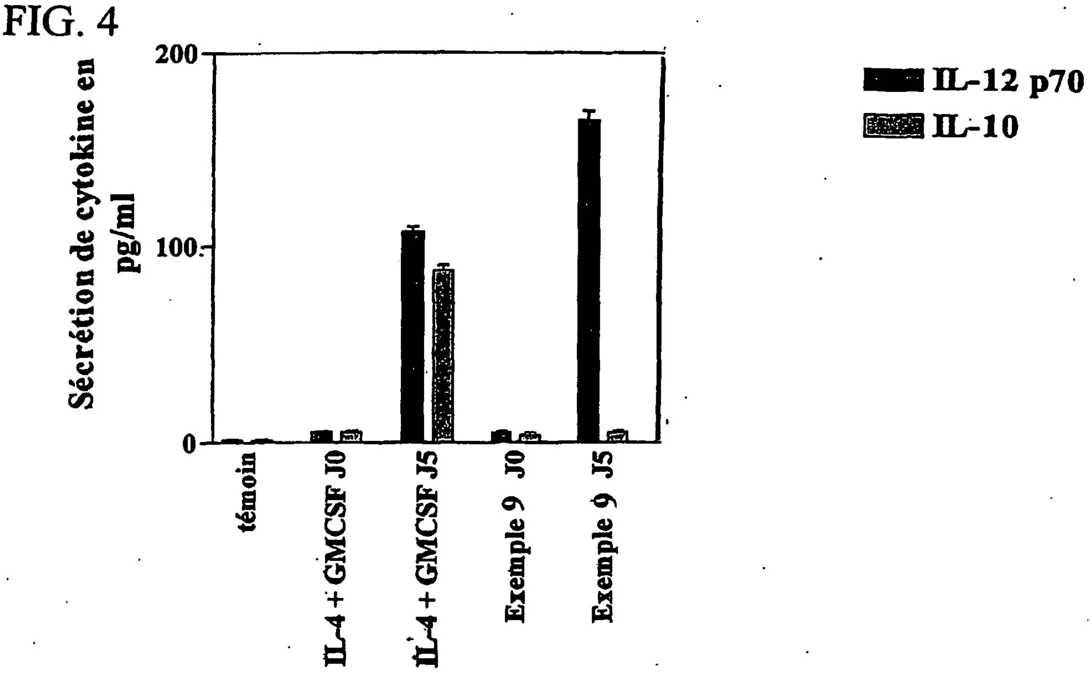
200
100
0
Sécrétion de cytokine en pg/ml
IL-12 p70
IL-10
témoin
IL-4 + GMCSF J0
IL-4 + GMCSF J5
Exemple 9 J0
Exemple 9 J5

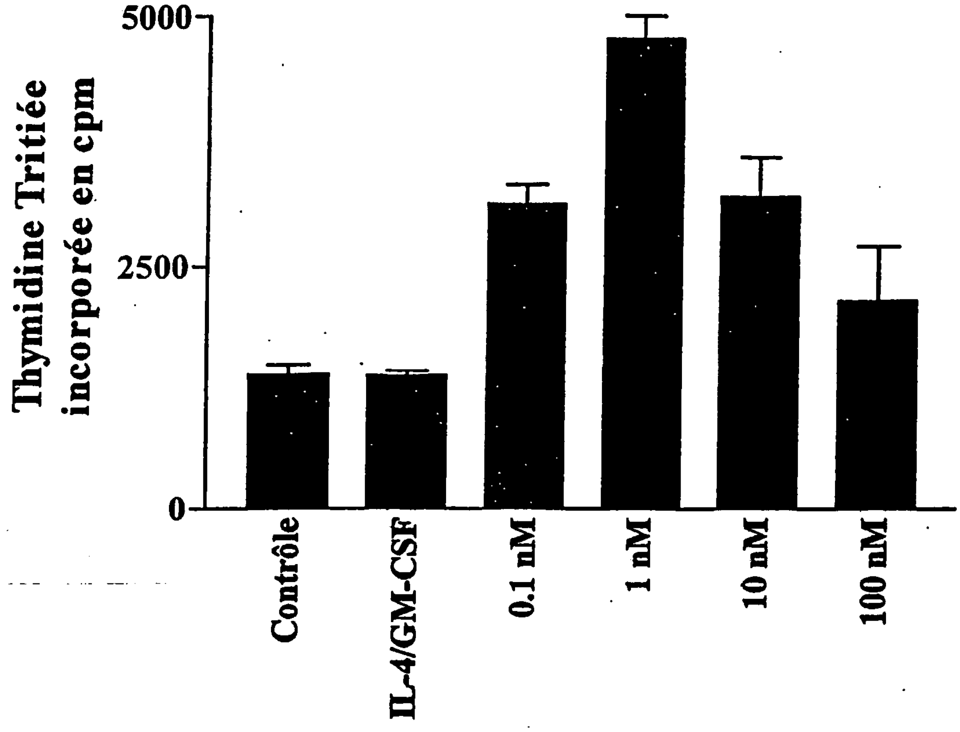
Thymidine Tritiée incorporée en cpm
5000
2500
0
Contrôle
IL-4/GM-CSF
0.1 nM
1 nM
10 nM
100 nM

FIG. 5

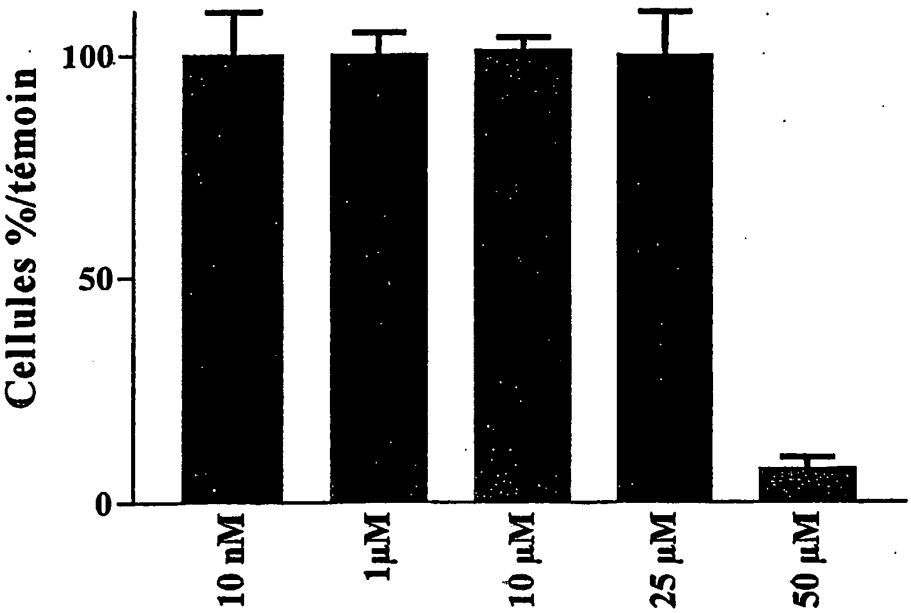
Cellules %/témoin
100
50
0
10 nM
1µM
10 µM
25 µM
50 µM

FIG. 6

FIG. 7

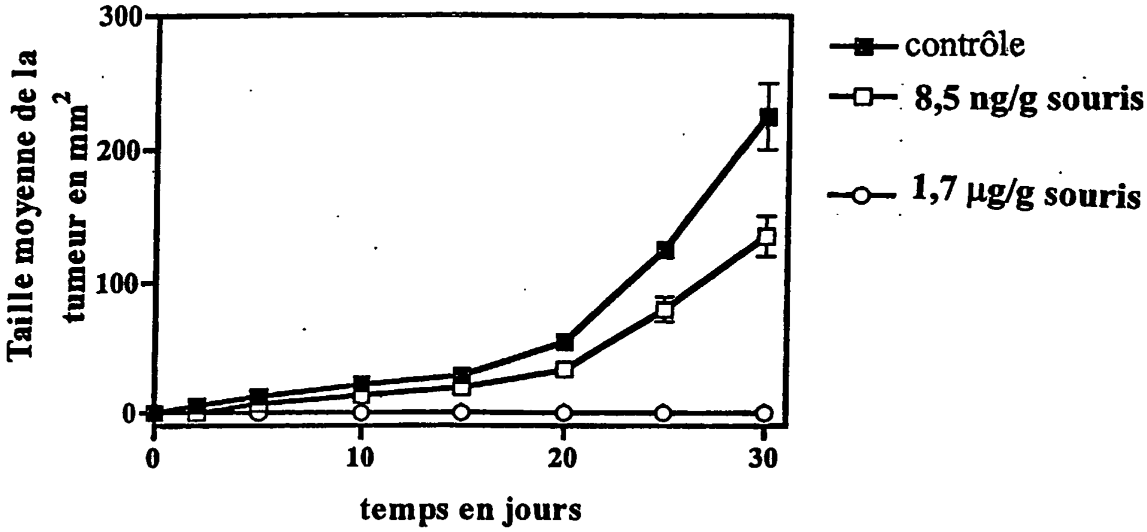
Taille moyenne de la tumeur en mm²
300
200
100
0
0
10
20
30
temps en jours
contrôle
8,5 ng/g souris
1,7 µg/g souris

FIG. 8

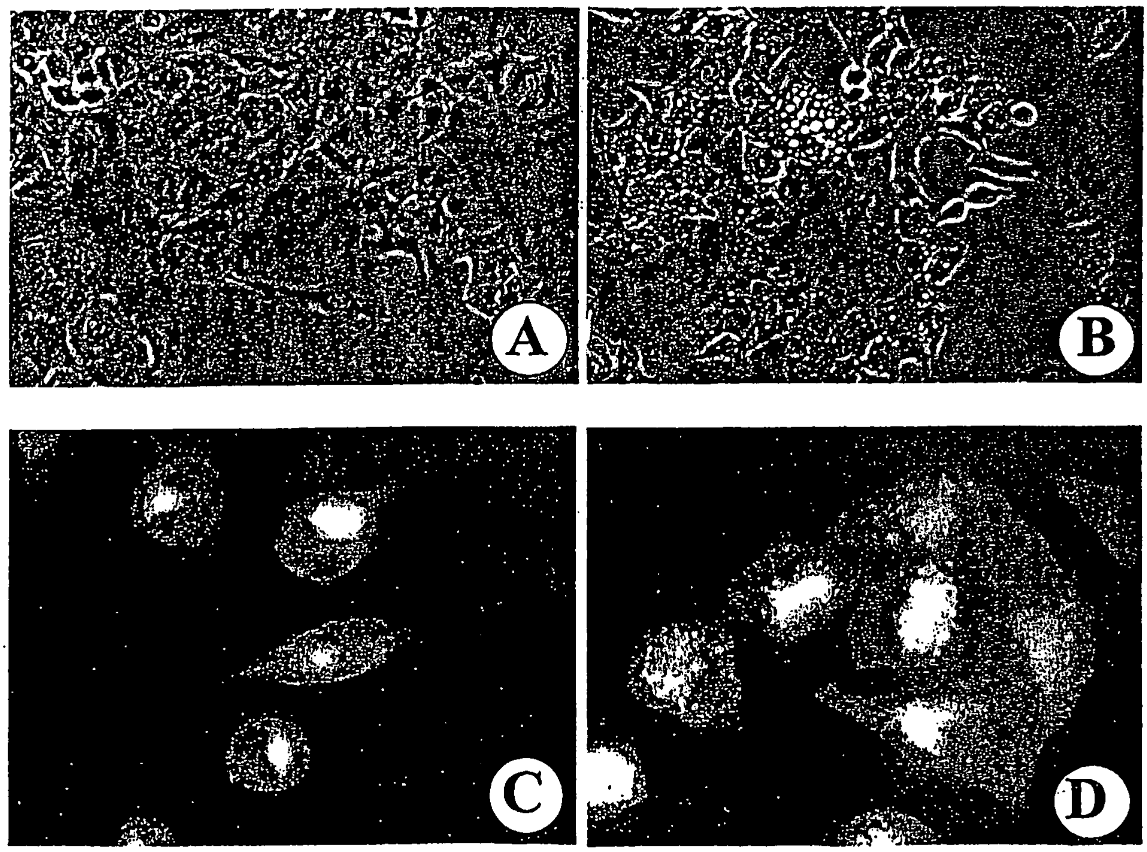
A
B
C
D

FIG. 9

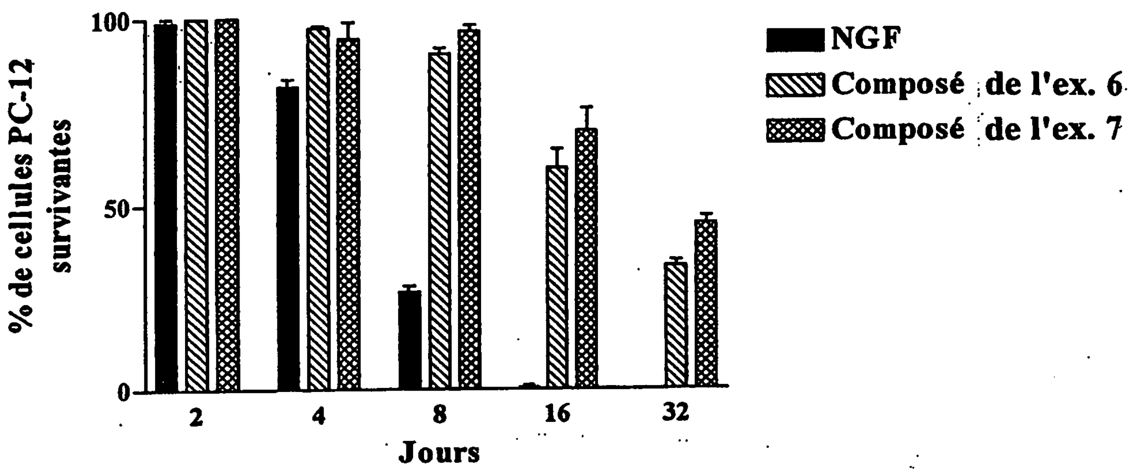
% de cellules PC-12 survivantes
100
50
0
2
4
8
16
32
Jours
NGF
Composé de l'ex. 6
Composé de l'ex. 7

FIG. 10

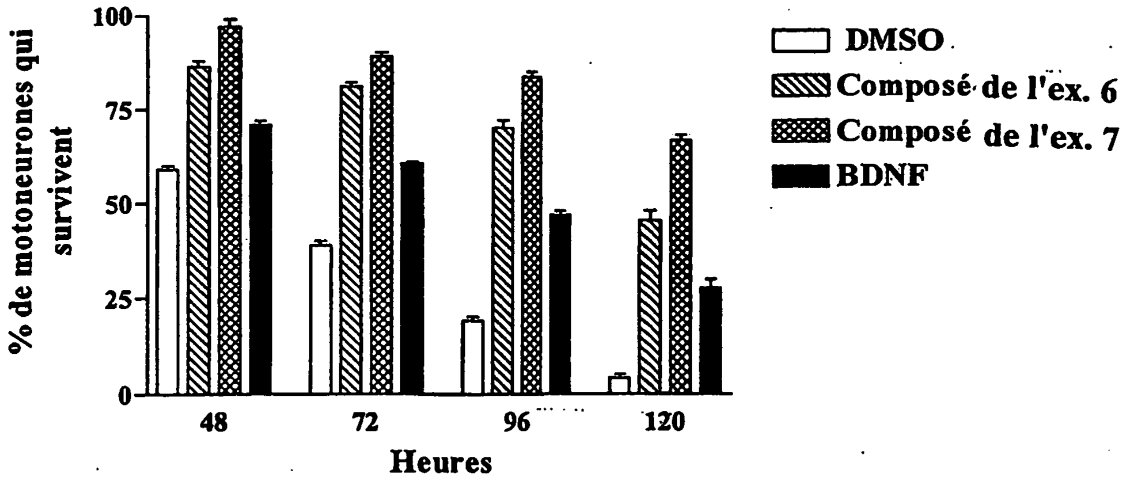
% de motoneurones qui survivent
100
75
50
25
0
48
72
96
120
Heures
DMSO
Composé de l'ex. 6
Composé de l'ex. 7
BDNF

FIG. 11

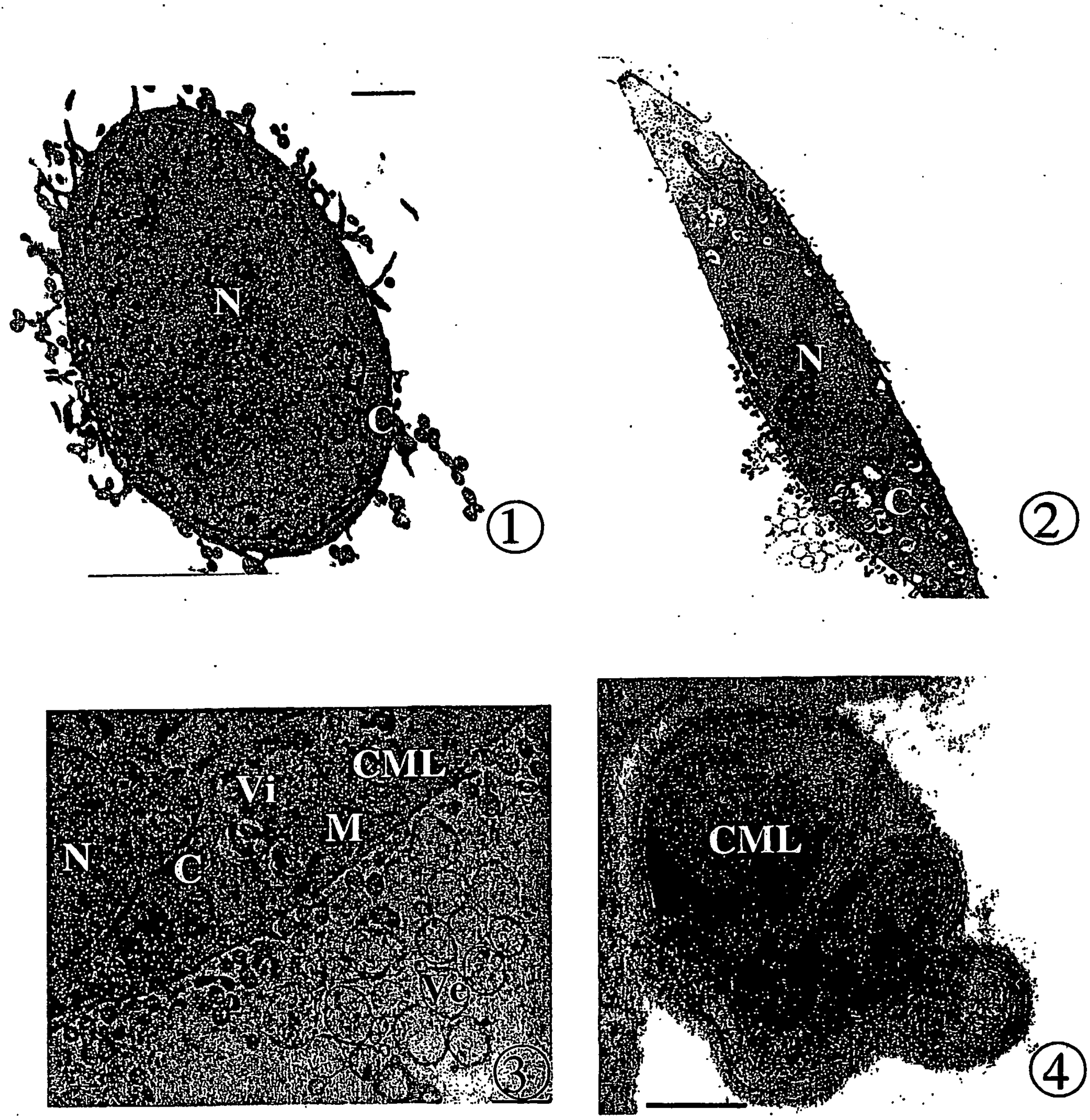

FIG. 12

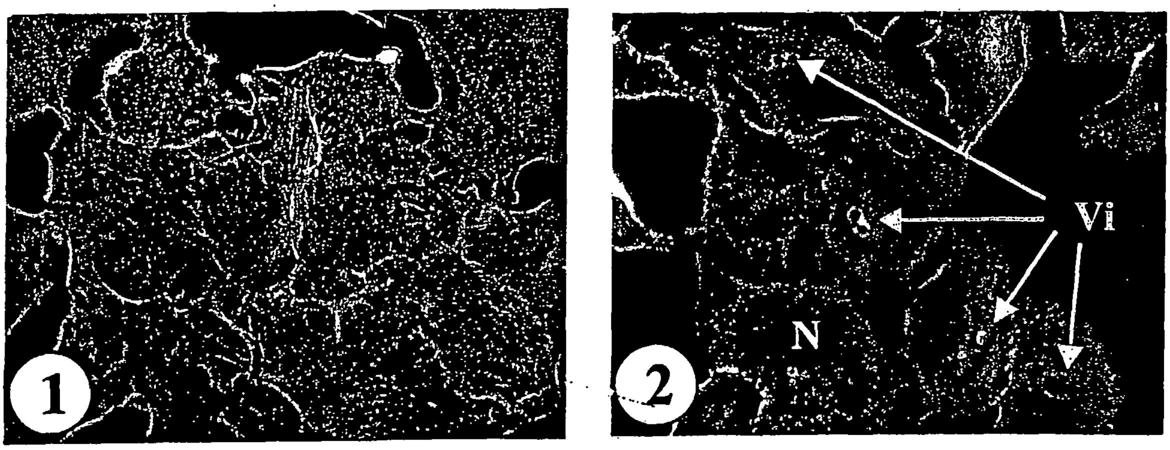


FIG. 13

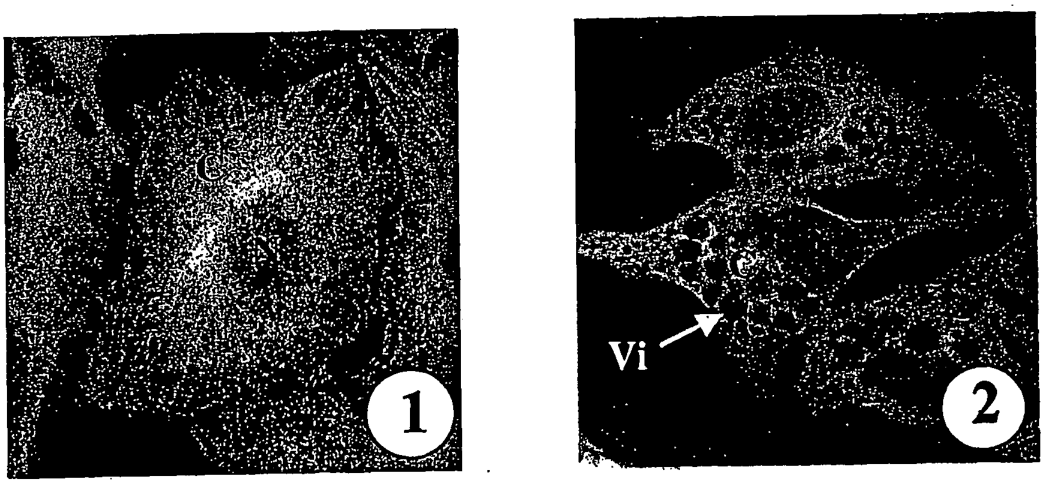

FIG. 14

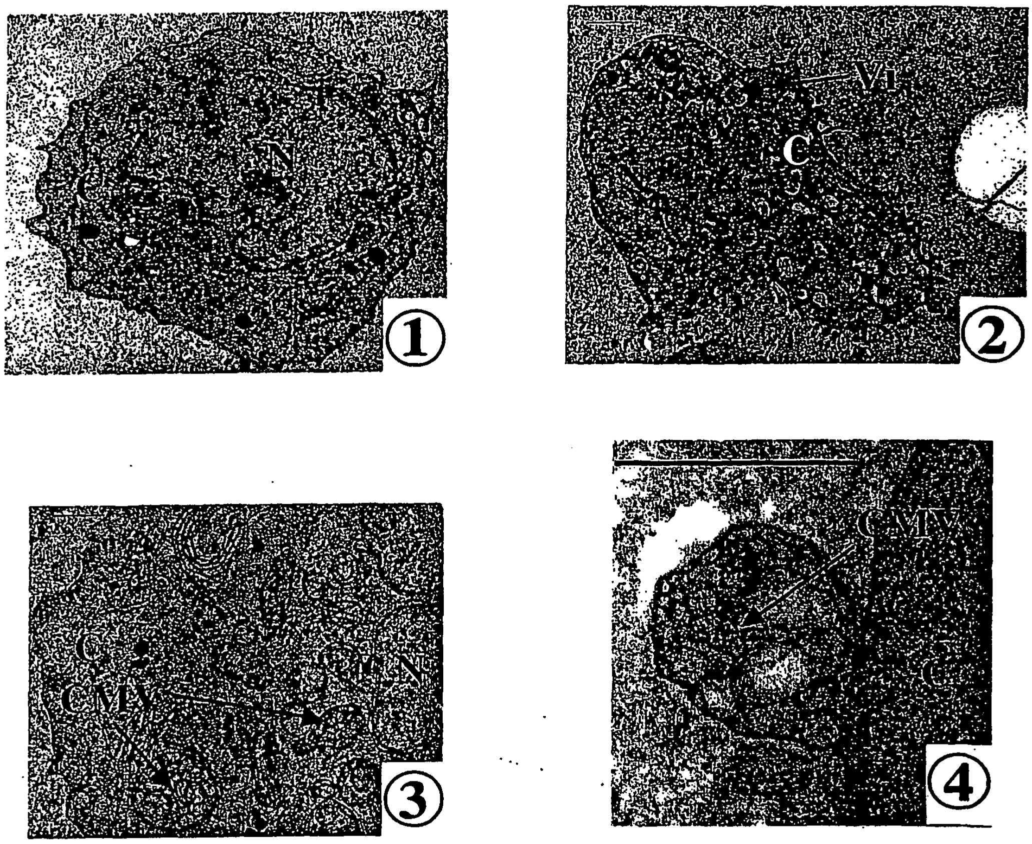


FIG. 15

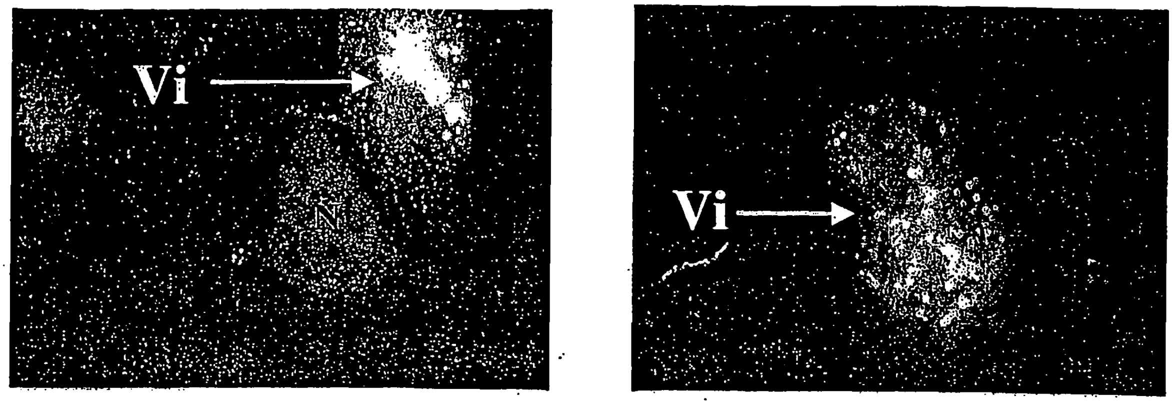

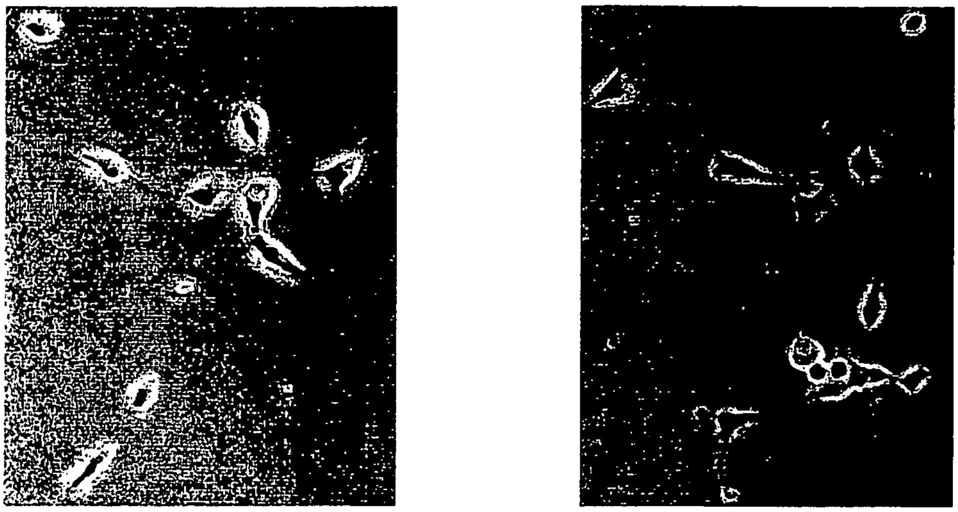

1 2

FIG. 16

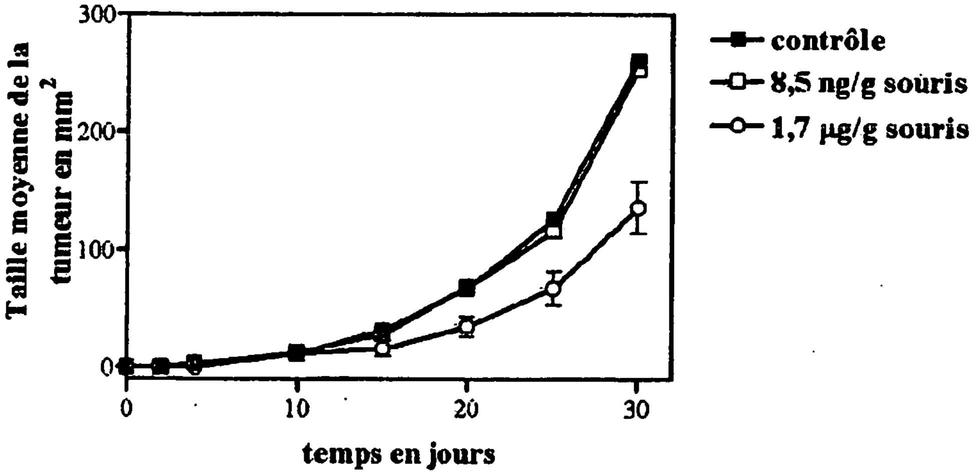


FIG. 17

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2047880


### Littérature non-brevet citée dans la description


- **MARTINEZ-ARCA S.** *J. Neurosci,* 2001, vol. 21 (11), 3830-38
- **DENZER K.** *J. Cell. Sci.,* 2000, vol. 113 (19), 3365-74
- **SCHULER P. ; STEINMAN RM.** *J. Exp. Med.,* 1997, vol. 8, 1183-7
- **ANGEVIN, ANDRÉ.** *Zitvogel, Bull Cancer,* 2000, vol. 87 (1), 107-15
- **LAWRENCE FONG ; EDGAR G. ENGLEMAN.** *Annu. Rev. Immunol.,* 2000, vol. 18, 245-273
- **PRADINES.** *J. Neurochem.,* 1995, vol. 64, 1954-64
- *Synthetic Communications,* 1997, vol. 27 (11), 1951-1962
- *Arzneimittel-Forschung,* 1995, vol. 45 (2), 190-4
- *Anticancer Research,* 1999, vol. 19 (2A), 1229-1234
- *J. Cell. Biol.,* Août 1982, vol. 94 (2), 253-262
- *Nature,* 09 Septembre 1982, vol. 299 (58 79), 165-167
- **DUONG.** *British Journal of Pharmacology,* 1999, vol. 28, 1385-1392
- *Amyotroph Lateral Scler Other Motor Neuron Discord,* 02 Mars 2001, vol. 1, 55-68
- *Brain Research Review,* 2000, vol. 3, 199-227